# EUROPÄISCHE PATENTSCHRIFT

(11) **EP 3 912 663 B1**
(45) Veröffentlichungstag und Bekanntmachung des Hinweises auf die Patenterteilung: **28.08.2024**
(21) Anmeldenummer: 21020311.3
(22) Anmeldetag: 10.07.2015
(51) Int. Cl.: A61M 16/00

(54) **BEATMUNGSGERÄT UND STEUEREINHEIT ZUR STEUERUNG EINER ATEMGASQUELLE**
ARTIFICIAL RESPIRATION DEVICE AND CONTROL UNIT FOR CONTROLLING A RESPIRATION GAS SOURCE
APPAREIL RESPIRATOIRE ET UNITÉ DE COMMANDE PERMETTANT DE COMMANDER UNE SOURCE DE GAZ RESPIRATOIRE

(30) Priorität: 05.09.2014 DE 102014012907; 15.09.2014 DE 102014011951
(43) Veröffentlichungstag der Anmeldung: 24.11.2021
(62) Teilanmeldung aus: 15002068.3
(73) Patentinhaber: Löwenstein Medical Technology S.A., 2557 Luxembourg (LU)
(72) Erfinder: SCHWAIBOLD, Matthias, 76228 Karlsruhe (DE)
(74) Vertreter: Marx, Thomas

(56) Entgegenhaltungen:
- WO-A1-2013/120690
- DE-A1- 19 516 536
- DE-B3- 102006 048 680
- US-A1- 2008 020 525

## Beschreibung

### Beatmungsgerät und Steuereinheit zur Steuerung einer Atemgasquelle

Die vorliegende Erfindung betrifft eine Steuereinheit zur Steuerung einer Atemgasquelle und ein Beatmungsgerät mit einer Steuereinheit.

Schlafstörungen beruhen in der Regel auf einer obstruktiven Schlaf-Apnoe (Obstructive Sleep Anpea, OSA), bei der der Atemfluss vorübergehend entweder deutlich verringert (Hypopnoe) oder vollständig unterbrochen (Apnoe) wird. Für einen erholsamen Schlaf ist dieser Sauerstoffmangel jedoch abträglich. Es kommt zu Stresssituationen, die lang und mittelfristig das Herz schädigen können. Deshalb werden Patienten mit einer obstruktiven Schlafapnoe durch Schlaftherapiegeräte oder Heimbeatmungsgeräte zusätzlich beatmet.

Zur Therapierung der Schlafapnoe gibt es unterschiedliche Geräte, die verschiedene Therapien umsetzen können. Eine Standardtherapie ist die CPAP-Therapie (Continuous Positive Airway Pressure), bei der das Beatmungsgerät dem Patienten einen gegenüber dem atmosphären Druck erhöhten Druck applizieren. Der Patient atmet selbst. Das Gerät unterstützt also nur die Spontanatmung, wobei durch den dauerhaften Druck die Atemwege geöffnet werden. WO2013/120690 beschreibt ein Beatmungssystem und ein Steuereinheit, wobei der Spontanatemfrequenz eine Rolle spielt.

Eine weitere Therapie ist die APAP-Therapie (Automated Positive Airway Pressure). Hierbei wird der Druck nur während einer Atemstörung (Apnoe oder Hypopnoe) angeboten. Alternativ werden Patienten mit einer periodischen Atmung oder mit einer zentralen Apnoe bisher mit Geräten therapiert, die zwei verschiedene Drucklevel anbieten, die sogenannten Bilevel-Geräte. Diese Geräte stellen zwei Drucklevel zur Verfügung, einen höheren inspiratorischen Druck (IPAP) und einen geringeren exspiratorischen Druck (EPAP). Das Zurverfügungstellen des Drucks erfolgt nur während Perioden, die eine reduzierte oder fehlende Spontanatmung aufweisen. Hierdurch wird das Auftreten einer Sauerstoffsättigung oder von Arousals verhindert. Leiden die Patienten unter einer Cheyne-Stokes-Atmung, bei der ein periodisches An- und Abschwellen der Atemtiefe und des Abstands der einzelnen Atemzüge vorliegt, erfolgt als Therapieform eine adaptive Servoventilation, bei dem der Atemdruck bei jedem Atemzug automatisch antizyklisch reguliert wird, um eine Stabilisierung der Atmung durch eine variable Druckunterstützung herbeizuführen.

Diesen auf einer Druckkontrolle basierenden Therapieformen steht eine volumenkontrollierte Therapie gegenüber. Bei einer Volumenkompensation wird ein Sollvolumen oder Targetvolumen vorgegeben, das von dem Patienten erreicht werden soll, damit er ausreichend ventiliert.

Das Gerät misst jeweils das Atemvolumen des Patienten und erhöht bei Unterschreitung des Targetvolumens (Zielvolumen) den zu verabreichenden Druck, in der Regel den inspiratorischen Druck. Typische Einstellmöglichkeiten für derartige Geräte sind das Zielvolumen, ein Intervall für den inspiratorischen Druck und die Regelgeschwindigkeit.

Aufgabe der vorliegenden Erfindung ist es, ein verbessertes Beatmungsgerät und eine Steuereinheit zur Steuerung einer Atemgasquelle vorzuschlagen, welche auch bei komplexen Erkrankungen eine komfortable und robuste Beatmung eines Patienten gewährleistet.

Gelöst wird die vorliegende Aufgabe durch eine Steuereinheit mit den Merkmalen des Anspruchs 1 sowie mit einem Beatmungsgerät mit den Merkmalen des Anspruchs 20.

Im Rahmen der Erfindung wurde festgestellt, dass eine Therapie mit einer Volumenkompensation eine geeignete Behandlung für Patienten mit Schlafstörungen ist, die auf einer Hypoapnoe beruhen. Durch die Volumenkompensation wird sichergestellt, dass die Patienten ausreichend beatmet werden und ein hinreichend großes Atemvolumen zur Verfügung steht. Hierdurch werden gewünschte Sauerstoffsättigungen zuverlässig erzielt. Die Patienten empfinden eine derartige Therapie als komfortabel und den Schlaf als erholsam.

Es wurde weiterhin festgestellt, dass Geräte zur Volumenkompensation und zum Bereitstellen eines Sollwertes für ein Tidalvolumen (Atemvolumen) in der Regel nur wenige, feste Einstellmöglichkeiten bieten, wie oben dargestellt. Darüber hinaus sind weitere Geräte bekannt, die andere bewährte Regelalgorithmen verwenden, beispielsweise eine automatische Bilevel-Druckapplikation oder eine antizyklische Servoventilation. Diese sind für andere Therapien gut geeignet, wie beispielsweise für Obstruktionen der Atemwege oder eine Cheyne-Stokes-Störung

Im Rahmen der Erfindung wurde erkannt, dass eine Kombination aus einer Zielvolumen-Regelung, also einer Regelung des Tidalvolumens des Patienten, und einer Regelung einer weiteren für die Beatmung des Patienten charakteristischen Beatmungskenngröße große Vorteile aufweist. Unter einer Regelung einer für die Beatmung charakteristischen Beatmungskenngröße wird beispielsweise eine automatische Einatemdruck-Regelung verstanden, die eine Ausatmung und/oder eine Einatmung erkennt. Eine weitere Regelung ist eine automatische Anpassung des Ausatemdrucks, wobei die Druck-Anpassung sowohl nach oben als auch nach unten erfolgen kann. Das Ziel ist hierbei, Obstruktionen der Atemwege zu therapieren.

Weitere Regelungen, die mit der Regelung des Tidalvolumens erfindungsgemäß kombiniert werden können, ist eine automatische Einstellung der Beatmungsfrequenz, eine Regelung der antizyklischen Druckreaktion (antizyklische Servoventilation) oder die Regelung des Übergangsdrucks zwischen dem inspiratorischen und dem exspiratorischen Druck.

Es ist selbstverständlich auch möglich, neben der Regelung des Tidalvolumens mehrere der anderen Regelungen zu kombinieren, so dass nicht nur das Tidalvolumen geregelt wird, sondern auch eine automatische Beatmungsfrequenz und zusätzlich beispielsweise eine automatische Anpassung des Ausatemdrucks oder eine antizyklische Servoventilation erfolgt. Dabei sind gleichzeitig beliebige Kombinationen der gegebenen und bekannten Regelungen mit der Regelung des Tidalvolumens denkbar. Die gleichzeitige Regelung des Tidalvolumens und einer weiteren Beatmungskenngröße bedeutet nicht die zeitgleiche Regelung sondern nur die Möglichkeit beide Größen gemeinsam zu regeln, um eine möglichst effiziente und robuste sowie für den Patienten komfortable und sichere Beatmung zu garantieren. Die Regelung kann zeitlich versetzt erfolgen, zeitlich überlappend oder zeitgleich. Das Beatmungsgerät sieht wenigstens zwei Regelungsmöglichkeiten in einer oder mehreren Steuereinheiten vor, wobei jedenfalls das Tidalvolumen geregelt wird.

Das Tidalvolumen wird bei der Regelung entweder pro Atemzug betrachtet, gemessen und geregelt oder pro Minute oder über mehrere Atemzüge hinweg, so dass erst nach einer vordefinierten Anzahl von Atemzügen das Tidalvolumen eingeregelt sein soll. Dazu wird in der Regel der Mittelwert über die vorgegebene Anzahl von Atemzügen betrachtet und ausgewertet. Besonders bevorzugt ist die Mittelwertbildung eine gewichtete Mittelung.

Die Regelung des Tidalvolumens erfolgt dadurch, dass zunächst der Gasstrom, der Volumenstrom oder eine äquivalente Atemgasgröße gemessen wird. Aus dieser Messgröße wird das von dem Patienten empfangene Luftvolumen bestimmt. Ein vordefinierter Sollwert des Tidalvolumens wird als Regelgröße verwendet. Der Sollwert (Soll-Tidalvolumen auch Zielvolumen oder Targetvolumen genannt) wird in der Regel in Milliliter angegeben. Seine Einstellung kann durch den Patienten selbst erfolgen. Die Größe des Zielvolumens kann durch einzelne Parameter von dem Gerät berechnet oder geschätzt werden, beispielsweise aus dem Alter, dem Gewicht und der Größe des Patienten oder aus ähnlichen Werten. Letztlich ist es auch möglich, diesen Wert durch Messungen, z.B. während einer Monitoringphase durch einen Arzt, zu ermitteln.

In Abhängigkeit von dem ermittelten Luftvolumen des Patienten und dem Zielvolumen (Soll-Tidalvolumen) erfolgt eine Regelung des inspiratorischen Drucks derart, dass der Sollwert des Tidalvolumens erreicht wird. Hierbei ist eine Regelung schon nach Messen des Luftvolumens bei einem Atemzug möglich oder über eine längere Zeitdauer, die mehrere Atemzüge des Patienten umfasst. Entweder kann eine vorgegebene Anzahl von Atemzügen beobachtet und gemessen werden oder es wird das Atemminutenvolumen des Patienten ermittelt. In diesem Fall erfolgt eine Regelung des Atemminutenvolumens im Vergleich zu einem auf die Minute bezogenen Soll-Tidalvolumens. Selbstverständlich können durch entsprechende Vorgabe an dem Beatmungsgerät und durch eine entsprechende Steuerung auch größere oder kleinere Zeiträume als Grundlage für die Regelung dienen.

Während bei den bisher vorhandenen Automatikmodi und Regelungen der bekannten Geräte, beispielsweise einer Auto-Bilevel-Regelung oder einer Regelung der antizyklischen Servoventilation kein Ziel-Tidalvolumen berücksichtigt wird, werden die Vorteile der unterschiedlichen Technologien gemäß der Erfindung geschickt kombiniert, um therapeutische Erfolge herbeizuführen.

Als ein Anwendungsbeispiel sei das Obesitas Hypoventilationssyndrom (OHS) angeführt, bei dem es zu einer Verengung der Atemwege durch das eigene Körpergewicht oder eine extreme Fettleibigkeit kommt. Obstruktionen der oberen Atemwege und Hypoventilationen treten häufig gemeinsam nur in der Rückenlage auf. Befindet sich der Patient in Seitenlage, können die Therapiedrücke, die dem Patienten verabreicht werden, deutlich abgesenkt werden. Dies führt zu einer Vergrößerung des Komforts und zu einer Verringerung der Nebenwirkungen. Erst wenn sich der Patient auf den Rücken dreht, wird automatisch auch der exspiratorische Druck (EPAP) angehoben, da das Beatmungsgerät die Obstruktionen selbst erkennt. Des Weiteren wird in der Rückenlage durch die auftretenden Obstruktionen das Mindestatemvolumen nicht mehr erreicht, so dass es zu einer fehlenden Sauerstoffsättigung bei dem Patienten kommt. Um das fehlende Mindestatemvolumen (das dem eingestellten Sollwert des Tidalvolumens entspricht) zu erzielen, erfolgt eine Regelung, bei der auch die Druckdifferenz zwischen inspiratorischem und exspiratorischem Druck angehoben wird. In Summe erfolgt somit eine stärkere Anhebung des inspiratorischen Drucks (IPAP) als des exspiratorischen Drucks (EPAP), der aber ebenfalls angehoben wird. Somit ist es erfindungsgemäß möglich, beide Symptome gleichzeitig erfolgreich zu therapieren.

Als weiteres Anwendungsbeispiel sei die Therapie bei vorhandenem COPD genannt (Chronic Obstructive Pulmonary Disease). Für eine Therapieeinstellung dieser chronisch obstruktiven Lungenerkrankung können die notwendigen Therapiedrücke nicht über viele Stunden hinweg durch geschultes Fachpersonal manuell eingestellt werden. Vielmehr muss der Prozess bei der Therapieeinstellung vereinfacht werden. Gemäß der erfinderischen Steuereinheit und dem entsprechenden Beatmungsgerät werden statt der dauerhaften Überwachung durch Fachpersonal nur die Druckgrenzen eingestellt, die der Patient noch toleriert. Darüber hinaus wird das Therapieziel vorgegeben, beispielsweise eine Obstruktionsvermeidung und die Erreichung eines Mindest-Tidalvolumens (Soll-Tidalvolumen). Die zur Therapie notwendigen Drücke werden bevorzugt von dem Gerät selbstständig ermittelt, wobei die Regelung derart abgestimmt ist, dass der Sollwert des Tidalvolumens eingeregelt wird und die Grenzwerte für die verwendeten Drücke eingehalten werden. Das Gerät kann beispielsweise automatisch die Beatmungsfrequenz anpassen und zusätzliche mandatorische Atemzüge erzeugen.

Für die beiden genannten beispielhaften Anwendungsfälle ergeben sich durch die erfindungsgemäße Steuereinheit und das Beatmungsgerät weitere Vorteile aufgrund der bevorzugten automatischen Einstellung der bedarfsgerechten Therapiedrücke. Ändern sich die Randbedingungen für die Therapie und Regelung, beispielsweise durch eine Erkrankung des Patienten, durch eine Veränderung seines Gewichts, durch Medikamenteneinnahme oder durch Alkoholkonsum, können die Therapiedrücke bedarfsgerecht angepasst und so eine optimale Therapie erzielt werden.

In einer bevorzugten Ausführungsform der Steuereinheit wird ein exspiratorischer Druck erzeugt, der geringer als der inspiratorische Druck ist, so dass eine Druckdifferenz gebildet wird. Bevorzugt gelten für den inspiratorischen wie für den exspiratorischen Druck jeweils eine untere und eine obere Druckgrenze, innerhalb derer der Druck jeweils veränderbar ist.

In einer bevorzugten Ausführungsform der Erfindung wird die Druckdifferenz geregelt, bis das von dem Patienten empfangene Luftvolumen gleich dem Sollwert des Tidalvolumens ist. Die Angleichung des aktuellen Luftvolumens an das Soll-Tidalvolumen erfolgt entweder über einen oder mehrere Atemzüge des Patienten oder über das Atemminutenvolumen des Patienten. Bevorzugt wird die mandatorische Beatmung des Patienten auch hier auf eine vorhandene Spontanatmung des Patienten abgestimmt, so dass mit der Einatmung des Patienten der inspiratorische Druck erzeugt und über eine Atemmaske verabreicht wird und bei einer Ausatmung der exspiratorische Druck an der Atemmaske anliegt.

Vorzugsweise wird von der Steuereinheit des Beatmungsgeräts durch zyklische Messung und Auswertung eines oder mehrerer Atemparameter eine Einatmung und eine Ausatmung erkannt. Aus der erkannten Einatmung und Ausatmung wird das momentane Tidalvolumen des Patienten gemessen und mit dem Sollwert des Tidalvolumens verglichen. Bei einer Abweichung des momentanen oder über eine vorgegebene Zeit oder Anzahl von Atemzügen gemittelte Tidalvolumen wird mit dem entsprechenden Soll-Tidalvolumen verglichen und bei einer Abweichung der Druck angepasst. Insbesondere bei einer Unterschreitung des Sollwertes wird der inspiratorische Druck oder die Druckdifferenz zwischen dem inspiratorischen und dem exspiratorischen Druck erhöht. Die Änderung des inspiratorischen Drucks und/oder der Druckdifferenz erfolgt bevorzugt innerhalb der vorgegebenen Druckgrenzen, sofern diese vorhanden sind.

Vorzugsweise erfolgt das Erkennen einer Einatmung und/oder Ausatmung des Patienten automatisch anhand von patientenspezifischen oder gerätespezifischen Kenngrößen, die bevorzugt einstellbar sind. Patientenbezogene Kenngrößen können hierbei beispielsweise das typische Volumen, der typische Atemfluss, die typische Frequenz, eine typische Inspirationsdauer oder Exspirationsdauer sein. Die typischen Werte können entweder durch Messung und Beobachtung, also aus der Historie, entnommen werden oder können als vordefinierte Werte hinterlegt sein. Als gerätespezifische Kenngrößen kann die Gesamtleckage des Systems, also die gewollte Leckage an der Atemmaske oder eine ungewollte Leckage durch einen schlechten oder fehlerhaften Sitz der Atemmaske verwendet werden. Auch kann aus dem aktuellen Druckverlauf auf die automatische Triggerung, d.h. die Empfindlichkeit zur Erkennung der Ein- und Ausatmung rückgeschlossen werden.

Vorzugsweise wird der exspiratorische Druck automatisch anhand eines vorgegebenen Therapiezieles angepasst. Soll beispielsweise der Komfort erhöht werden, ist eher ein geringerer exspiratorischer Druck zu wählen. Eine Anpassung des Ausatemdrucks kann sowohl nach oben als auch nach unten erfolgen. Ziele, die durch die Anpassung verfolgt werden, sind beispielsweise die Therapie von Obstruktionen der Atemwege, die Steigerung des Cardiac-Outputs, die Optimierung des Atemminutenvolumens oder die Redaktion von Nebenwirkungen neben der bereits erwähnten Steigerung des Komforts.

Bevorzugt erfolgt die Einstellung innerhalb von Druckgrenzen für den exspiratorischen Druck. Daneben sind in der Regel Druckgrenzen für die minimale und maximale Druckdifferenz einstellbar. Die Drücke für den inspiratorischen Druck ergeben sich dann rechnerisch und können in einem Beatmungsgerät ermittelt, dargestellt und/oder gespeichert werden.

Die Kombination aus der automatischen Anpassung des exspiratorischen Drucks mit einer Regelung des Tidalvolumen bietet insbesondere Vorteile bei OHS-Patienten (Obesitas Hypoventilationssyndrom-Patienten). Durch die Kombination kann hier der Titrationsaufwand zum Auffinden der konkreten Drücke erheblich reduziert werden. Darüber hinaus ermöglicht die Erfindung eine laufende Anpassung an den Therapiebedarf des Patienten, der sich entweder langsam verändern kann, z. B. beim Krankheitsverlauf oder bei einer Gewichtsänderung, oder schnell, beispielsweise durch Lagewechsel des Patienten in der Nacht. Darüber hinaus ändert sich normalerweise auch sowohl der Bedarf des Druckes zur Schienung der oberen Atemwege, also die Anpassung des exspiratorischen Drucks gegen die Obstruktionen, als auch der Bedarf nach Druckunterstützung, nämlich die Änderung der Druckdifferenz (IPAP minus EPAP) gegen Hypoventilation.

In einer bevorzugten Ausführungsform wird neben der Änderung des Tidalvolumens auch die Beatmungsfrequenz für die mandatorische Beatmung geregelt. Bevorzugt erfolgt bei dieser Art der mandatorischen Beatmung eine automatische Regelung der Beatmungsfrequenz in Abhängigkeit von der Spontanfrequenz des Patienten, die vom Gerät ermittelt wird. Bevorzugt wird der Abstand der mandatorischen Atemzüge zueinander und zum letzten spontanten Atemzug selbständig bestimmt. Bespielsweise kann das Beatmungsgerät, das die erfinderische Steuereinheit verwendet, die mandatorische Atemfrequenz als Prozentsatz der Spontanatemfrequenz oder als festes Delta zur Spontanatemfrequenz, also mit einer festen Differenz, bestimmt werden. Alternativ ist auch eine iterative Beschleunigung oder Verlangsamung der Frequenz möglich, mit dem Ziel der Erreichung einer höheren oder niedrigeren Quote an spontanen Atemzügen. Eine automatische Frequenzregelung in Kombination mit der Regelung des Tidalvolumens ermöglicht also insgesamt eine bessere Regelung des Atemzeitvolumens bzw. des Atemminutenvolumens. Darüber hinaus ist auch zusätzlich oder alternativ eine Minimierung der Atemanstrengung als Therapieziel verwirklichbar.

Bei einer Kombination der Regelung des Tidalvolumens mit einer Regelung der antizyklischen Servoventilation kann der Sollwert des Tidalvolumens als ein unterer Grenzwert hinterlegt sein. Bevorzugt erfolgt bei einem Tidalvolumen oberhalb des Grenzwertes, also oberhalb des Soll-Tidalvolumens eine zusätzliche antizyklische Druckreaktion, um Schwankungen des Tidalvolumens entgegenzuwirken. Dabei kann beispielsweise entweder ein längerer Durchschnitt des Atemvolumens gebildet und dann ein vorgegebener prozentualer Anteil (beispielsweise 95 % oder 90 %) als Zielwert übernommen werden. Alternativ kann einer Schwankung der Atmung dadurch entgegengewirkt werden, dass bei fallendem Volumen mit stärkender Druckunterstützung reagiert wird; bei steigendem Tidalvolumen wird die Druckunterstützung reduziert.

Bei herkömmlichen Geräten, die ein Verfahren zur reinen Servoventilation anbieten, wird kein Tidalvolumen oder Soll-Tidalvolumen vorgegeben. Zwar finden die Geräte das Zielvolumen in der Regel automatisch, allerdings ist dies nur möglich, wenn das durchschnittliche Volumen des Patienten ausreichend groß ist, wie bei einer reinen periodischen Atmung. Entsteht dagegen zusätzlich eine respiratorische Insuffizienz, wie es häufig bei Herzpatienten als Lungenödem vorliegt, kann auch die mittlere Atmung nicht ausreichend zur Erzielung guter Blutgaswerte sein. Durch die Kombination mit einem fest vorgegebenen Soll-Tidalvolumen wird somit eine Untergrenze für das automatisch von der Servoventilation gefundene Volumen festgelegt, ab der die Druck- und gegebenenfalls auch die Frequenzunterstützung in jedem Fall erhöht wird.

In einer bevorzugten Ausführungsform wird die applizierte Druckdifferenz zwischen dem inspiratorischen Druck und dem exspiratorischen Druck in Abhängigkeit der Volumendifferenz zwischen dem aktuell gemessenen Tidalvolumen des Patienten und dem vorgegebenen Soll-Tidalvolumen verändert und angepasst. Besonders bevorzugt kann eine adaptive Anpassung erfolgen.

Bei bekannten Tidalvolumen-Regelungen ändert sich die Druckdifferenz. In der Regel wird dabei eine Rampensteilheit für die Änderung der Differenz pro Sekunde angegeben. Bei einer fest eingestellten zeitlichen Druckänderung wird das Zielvolumen (Soll-Tidalvolumen) bei einer kleineren Druckdifferenz früher erzielt als bei einer größeren Druckdifferenz. In diesem Fall kann unter bestimmten Umständen sogar das Erreichen des Sollwertes ausgeschlossen sein. Eine derartig starre Anpassung reduziert nicht nur den Komfort für den Patienten, sondern auch die Beatmungseffektivität.

Erfindungsgemäß wird besonders bevorzugt die Steigung des Druckanstiegs adaptiv an die Druckdifferenz angepasst. Dies kann beispielsweise durch eine vorgegebene Regelzeit sein, innerhalb der der Druck angepasst sein muss. Eine derartige Zeitspanne wird auch als Rampenzeit bezeichnet. Alternativ kann ein prozentualer Anteil der Inspiration und Exspiration verwendet werden. Vorteilhaft ist hierbei, dass sich die Druckdifferenz, die Atemfrequenz und das Inspirations- zu Exspirationsverhältnis des Patienten automatisch anpassen. Als weitere Möglichkeit kann eine Steuerung über ein vorgegebenes Druckprofil erfolgen. Beispielsweise ist es möglich, ein Druckprofil über mehrere Stützstellen anzugeben. Das Profil ist bevorzugt derart ausgebildet, dass der resultierende Solldruckverlauf der Atemflusskontur des Patienten folgt.

In einer bevorzugten Ausführungsform erlaubt die erfinderische Steuereinheit die Überwachung des Erreichens des Sollwertes des Tidalvolumens. Bevorzugt kann ein Alarmsignal erzeugt und ausgegeben werden. Ein derartiges Signal ist beispielsweise ein Tonsignal oder ein optisches Signal

In einer bevorzugten Ausführungsform erfolgt die Bestimmung des Tidalvolumens über eine Messung des inspiratorischen Gasflusses des Patienten, der über die Bestimmung des von dem Gerät abgegebenen Gesamtgasflusses und des über eine vorhandene Leckage abgegebenen Gasflusses, des sogenannten Leckagegasflusses, ermittelt werden. In einer besonders bevorzugten Ausführungsform kann zur Bestimmung des Tidalvolumens auch das "Totraumvolumen" des Patienten berücksichtigt werden, also die nicht für den Gasaustausch verwendeten Bereiche, wie beispielsweise Luftröhre und obere Atemwege, usw..

An die Berechnung des Tidalvolumens werden hohe Anforderungen gestellt, da die korrekte Bestimmung des Tidalvolumens die wichtigste Voraussetzung für eine möglichst genaue und fehlerfreie Regelung des Volumens ist. Bevorzugt sollte diese Abweichung kleiner 20 % oder kleiner 15 % sein. Zur Bestimmung des Volumens können die Zeiträume von Inspiration und Exspiration aus dem Verlauf des Patientenflows bestimmt werden. Bevorzugt wird entweder der inspiratorische Patientenflow oder der exspiratorische Patientenflow aufsummiert über eine vorgegebene Zeit und anschließend entsprechend gemittelt. Es ist aber auch möglich, nur über einen Atemzug den Flow (Gasfluss) zu messen. Um den Flow sehr genau messen zu können, subtrahiert das Gerät von dem sogenannten Geräteflow, die gewollte und ungewollte Leckage. Der Geräteflow ist der Gesamtgasfluss, der von dem Gerät in dem Beatmungsschlauch abgegeben wird. Die gewollte Leckage lässt sich beispielsweise aus den abgegebenen Therapiedrücken und der Maskenkennlinie ermitteln, die oft als Ausgangsflow über den Druck dargestellt ist. Die ungewollte Leckage, z. B. ein falsches Anlegen der Atemmaske, lässt sich z. B. durch Bildung der Differenz aus inspiratorischem und exspiratorischem Rohvolumen und der gewollten Leckage herleiten. Durch diese Maßnahmen kann das dem Patienten zur Verfügung gestellte Atemgasvolumen sehr genau berechnet werden.

Da für den Gasaustausch in der Lunge nur das alveoläre Volumen und nicht das von der Luftröhre und den oberen Atemwegen und anderen Körperteilen gebildete "Totraumvolumen" beiträgt, lässt sich eine effiziente Berechnung des relevanten Volumens beispielsweise durch eine Schätzung des "Totraumvolumens" bilden. Hierbei wird stets das Volumen pro Atemzug betrachtet. Je höher die Atemfrequenz ist, desto größer ist damit auch der Anteil an "Totraumvolumen" und desto kleiner ist das effektive für den Gasaustausch zur Verfügung stehende Volumen für den Patienten. Das Totraumvolumen kann beispielsweise aus der Körpergröße des Patienten geschätzt werden oder über eine CO₂-Messung ermittelt werden. Bevorzugt ist es möglich, das Todvolumen am Gerät direkt einzugeben, sofern es bekannt ist oder als festen Anteil des Tidalvolumens bei einer Normalatemfrequenz ermittelt werden.

Das erfindungsgemäße Beatmungsgerät umfasst eine Atemgasquelle zum Erzeugen eines Gasdrucks für einen Patienten, der mittels einer Atemmaske abgegeben wird. Das Gerät weist eine Messeinheit zum Messen einer für die Einatmung und Ausatmung charakteristischen Messgröße sowie eine Steuereinheit auf, die aus der Messgröße das Tidalvolumen des Patienten bestimmt. In der Steuereinheit wird der aktuelle Messwert des Tidalvolumens mit einem Sollwert des Tidalvolumens (Solltidalvolumen) verglichen. Entsprechend dem Vergleich erfolgt eine Regelung der Atemgasquelle derart, dass der erzeugte Gasdruck zur Verfügung gestellt wird bis der Sollwert des Tidalvolumens erreicht ist. Zusätzlich erfolgt eine weitere Regelung einer für die Beatmung des Patienten charakteristischen Beatmungskenngröße, wodurch ebenfalls ein entsprechendes Regelsignal an die Atemgasquelle abgegeben wird. Der Sollwert des Tidalvolumens kann fest vorgegeben sein oder entsprechend vorgegebener Parameter oder Messgrößen aktuell ermittelt und verändert werden.

Bespielsweise kann in dem Gerät oder auch gemäß der Steuereinheit das für einen Patient benötigte Soll-Tidalvolumen, das sowohl das Volumen für einen Atemzug wie auch ein Minutenvolumen sein kann, automatisch über eine Formel oder eine Tabelle ermittelt werden, die im Gerät hinterlegt sein können. Insbesondere erfolgt die Ermittlung des Soll-Tidalvolumens durch Eingabe von Körpergröße oder Körpergewicht oder einem für die eingegebene Körpergröße entsprechenden Idealgewicht des Patienten. Alternativ kann das Tidalvolumen oder Minutentidalvolumen (Minutenatemvolumen) in einer Lernphase automatisch aus den Blutgasen des Patienten ermittelt werden, beispielsweise durch vorübergehenden Anschluss eines Messgeräts oder eines Schätzgeräts für den Sauerstoff oder CO₂-Gehalt des Blutes.

Bevorzugt verfügt das Beatmungsgerät über eine Alarmierungseinheit, um ein Alarmsignal auszugeben. Verlassen das Tidalvolumen, das auf die Minute bezogene Tidalvolumen (Atemminutenvolumen) oder die Atemfrequenz mindestens einen der im Gerät beispielsweise einstellbaren Schwellwerte für eine vordefinierte Zeit, so wird ein Alarm ausgelöst. Insbesondere erfolgt eine Alarmierung, wenn das Soll-Tidalvolumen z.B. nach einer vordefinierten Zeitspanne nicht erreicht werden kann, obwohl eine Erhöhung der Druckunterstützung durch einen erhöhten inspiratorischen Druck oder eine erhöhte Druckdifferenz oder durch eine Erhöhung der Atemfrequenz erfolgt ist. Somit wird der Patient oder der Anwender des Gerätes, beispielsweise medizinisches Personal, über eine Veränderung im Krankheitsverlauf des Patienten hingewiesen oder auf eine unzureichende Wahl der Geräteeinstellungen aufmerksam gemacht. Vorzugsweise umfasst die Alarmierungseinheit dazu eine Alarmgebereinheit. Besonders bevorzugt ist ein Lautsprecher oder ein Buzzer vorgesehen. Alternativ oder zusätzlich kann ein optischer Alarm ausgegeben werden.

An einer Anzeigevorrichtung des Geräts werden Parameter der aktuellen Atmung des Patienten sowie Geräteeinstellungen ausgegeben. Bevorzugt umfasst die Anzeigeeinheit ein Display, um die Parameter oder Beatmungskenngrößen oder Messwerte oder aus Messwerten weiterverarbeitete Werte anzuzeigen. Insbesondere gehören hierzu das Tidalvolumen (Volumen pro Atemzug oder das Tidalminutenvolumen), die Beatmungsfrequenz, die inspiratorischen und exspiratorischen Drücke sowie die Grenzwerte für diese Drücke, das Zielvolumen oder Soll-Tidalvolumen, die Art der Druckunterstützung, beispielsweise die Druckdifferenz, oder auch eine Hintergrundfrequenz, die vorhanden sein kann, um eine mandatorische Beatmung an diese Frequenz anzupassen. Anhand dieser Parameter und Kenngrößen kann der Anwender, Patient oder Arzt die Geräteeinstellungen optimieren. Bevorzugt weist das Beatmungsgerät eine Schnittstelle auf, um die anzuzeigenden Werte an ein externes Anzeigegerät zu übertragen. Somit ist auch eine externe Speicherung der Daten möglich.

Zur Abspeicherung ist bevorzugt eine Speichereinheit vorgesehen, um die gemessenen Werte, die charakteristischen Beatmungskenngrößen, die Alarme, das Atemvolumen oder die Einstellparameter abzuspeichern. Eine optionale Recheneinheit dient zur statistischen Auswertung der Parameter. Selbstverständlich können auch andere Einstellparameter oder Atemcharakteristika aufgezeichnet, abgespeichert oder dargestellt werden. Gegebenenfalls können sie an einen Serverrechner übertragen werden, beispielsweise über eine kabelgebundene oder eine drahtlose Schnittstelle. Aus diesen Daten lassen sich dann mittels der Recheneinheit statistische Kennzahlen ermitteln, um beispielsweise die Wirksamkeit der Regelungen nachzuweisen und zu überprüfen. Selbstverständlich lassen sich auch die Erfolge einer längeren Therapie auswerten und bewerten. Neben den oben genannten Parametern sind auch Größen wie die Anzahl der Atemzüge, Dauer der Therapiephasen, prozentuale Dauer der Phasen mit Über- und Unterschreiten des Zielvolumens oder Soll-Tidalvolumens interessant und können verarbeitet, gespeichert oder dargestellt werden.

Die kabelgebundene oder eine drahtlose Schnittstelle ist innerhalb des Gerätes oder im Bereich des Gerätegehäuses vorgesehen. Alternativ kann über eine erste Schnittstelle ein Schnittstellenmodul, beispielsweise per Rastung, angesteckt werden, welches weitere Schnittstellen bereitstellt. Über die Schnittstelle kann eine Verbindung zu einem Server oder einem Netzwerk hergestellt werden, über die Daten aus dem Gerät ausgelesen, das Gerät konfiguriert oder eine Service-Funktion durchgeführt werden kann. Technologien können GSM, GPRS, UMTS, LTE, Sigfox, LOLA, Bluetooth oder ähnliche Funkstandards sein.

In einer bevorzugten Ausführungsform hat das Beatmungsgerät eine Bedieneinheit, die eine oder mehrere Mensch-Maschine-Schnittstellen aufweist und bevorzugt ein Touchscreen-Display hat. Über ein derartiges Display ist eine einfache benutzerfreundliche Bedienung des Geräts möglich. Darüber hinaus können zusätzlich oder alternativ auch Tasten, ein Drehrad oder ähnliche Schalter oder Schieber vorgesehen sein. Die Auswahl von einzustellenden Parametern kann beispielsweise über Auswahllisten oder Auswahltabellen oder sogenannte Menüs erfolgen, die auf dem Display angezeigt werden können. Insbesondere bei Touchscreen-Displays lässt sich eine Auswahl sehr einfach für den Patienten durchführen. Die Anzeige der einzelnen Parameter oder die Auswahl einzelner Parameter lassen sich in unterschiedlichen Anzeigeebenen oder unterschiedlichen Anzeigefeldern darstellen. Beispielsweise können die Parameter für die Beatmung mit einem Tidalvolumen und einem Sollwert des Tidalvolumens in einer anderen Menüebene oder in einem anderen Displaybereich dargestellt werden, wie reine Geräteparameter oder Serviceparameter, die beispielsweise auch mit einem Kennwort gesichert sein können.

In einer weiter bevorzugten Ausführungsform weist das Beatmungsgerät ein Display auf, das gegenüber der Standfläche des Geräts abgewinkelt ist. Bevorzugt ist die Neigung des Displays nicht rechtwinklig oder parallel zur Standfläche; der Neigungswinkel ist also weder 90 Grad noch Null Grad. Besonders bevorzugt ist das Display mit einem schrägen Winkel zwischen 20 Grad und 70 Grad gegenüber der Standfläche gekippt. Durch die Schrägstellung des Displays, insbesondere in dem bevorzugten Winkelbereich kann ein Ablesen der Parameter komfortabel im Liegen, Stehen oder Sitzen erfolgen. Besonders bevorzugt ist ein Display, das in seiner Neigung variabel ist und in der Regel zwischen Null Grad und 90 Grad verschwenkbar ist. Neben einem horizontalen Verschwenken kann auch ein vertikales Verschwenken vorteilhaft sein.

In einer besonders bevorzugten Ausführungsform kann die Helligkeit des Displays eingestellt werden. Insbesondere kann ein automatisches Dimmen der Displayhelligkeit erfolgen, beispielsweise nach einer gewissen Zeit seit der letzten Eingabe oder in einem vorgegebenen Bereich zwischen einer Minute und mehreren Minuten. Die Helligkeit des Displays kann automatisch an die Umgebungshelligkeit angepasst sein, damit der Patient ungestört schlafen kann.

Über eine Schnittstelle (drahtlos oder Stecker) kann zur Therapiekontrolle oder zum Lernen des korrekten Soll-Tidalvolumens bzw. Soll-Minutenvolumens bzw. Soll-Alveolären-Atemvolumens ein Pulsoximeter adaptiert werden. Alternativ oder ergänzend können auch CO2-Messgerätes, z. B. ein ptCO2-Messgerätes und/oder ein erweitertes Diagnosegerät angeschlossen werden. Das erweiterte Diagnosegerät registriert Effort, Körperlage, Pulsfrequenz, PTT, Pulswellenamplitude, autonome Arousals, Schlafstadien oder einen Indikator für eine Herzerkrankung.

Das erfindungsgemäße Beatmungsgerät umfasst eine Atemgasquelle zum Erzeugen eines Gasdrucks für einen Patienten, der mittels einer Atemmaske abgeben wird, eine Messeinheit zum Messen einer für die Einatmung oder Ausatmung charakteristischen Messgröße und eine Steuereinheit, die aus der Messgröße das Tidalvolumen des Patienten bestimmt und mit einem Sollwert des Tidalvolumens vergleicht und die Atemgasquelle derart regelt, dass der erzeugte Gasdruck zur Verfügung gestellt wird bis der Sollwert des Tidalvolumens erreicht ist, wobei zusätzlich eine weitere Regelung einer für die Beatmung des Patienten charakteristischen Beatmungskenngröße erfolgt und ein entsprechendes Regelsignal an die Atemgasquelle abgegeben wird. Erfindungsgemäß erfolgt die Regelung einer oder mehrerer für die Beatmung des Patienten charakteristischen Beatmungskenngrößen. Wobei die Beatmungskenngrößen einzelne Parameter wie Druck, Fluss oder Volumen, Frequenz oder auch komplexe Muster sein können.

Erfindungsgemäß erfolgt die Regelung des Tidalvolumens und/oder der Frequenz und/oder des EPAP und/oder des IPAP und/oder des PEEP und/oder von Rampen des EPAP und/oder von Rampen des IPAP und/oder der Inspirationszeit und/oder der Exspirationszeit und/oder des Exspirationsvolumens und/oder der Hustenunterstützung und/oder der antizyklischen Servoventilationsregelung. Beispielsweise werden EPAP und/oder PEEP so gewählt, dass Obstruktionen der oberen Atemwege vermieden werden und zudem der Sollwert für das Tidalvolumen erreicht wird.

Beispielsweise werden EPAP und/oder PEEP so gewählt, dass Obstruktionen der oberen Atemwege vermieden werden und zudem der Sollwert für das Tidalvolumen erreicht wird, wobei ebenfalls die Frequenz angepasst wird. Bei Absenkung des Tidalvolumens und somit des Minutenvolumens des Patienten, wird die Beatmungsfrequenz erhöht. Bei Anstieg des EPAP wird ebenfalls die Beatmungsfrequenz erhöht, um das Tidalvolumen zu erreichen.

Beispielsweise wird so zudem eine Steuercharakteristik mit einer Mindestexspirationszeit oder einem Mindestexspirationsvolumen vorgegeben. Dazu wird als Messgröße der Fluss oder der Druck oder das Volumen an Atemgas erfasst. Eine Druckerhöhung auf den IPAP-Druck wird so lange unterdrückt, bis das Mindestexspirationsvolumen oder die Mindestexspirationzeit erreicht ist.

Die Steuerung erhöht den Atemgasdruck in der Inspiration beispielsweise, wenn ein den Atemfluss oder das Atemzugvolumen repräsentierendes Signal anzeigt, dass der Exspirationsvorgang des Patienten nicht beendet ist.

Die Steuerung erhöht den Atemgasdruck in der Exspiration beispielsweise, wenn ein den Atemfluss oder das Atemzugvolumen repräsentierendes Signal anzeigt, dass der Exspirationsvorgang des Patienten nicht beendet ist.

Die Steuerung ist alternativ mit einer Triggersignalunterdrückung versehen, die eine Druckerhöhung für eine vorgebbare Blockadezeit sperrt, die mit einer exspiratiorischen Druckabsenkung beginnt.

Alternativ ist die Mindestexspirationszeit oder als Mindestexspirationsvolumen als Triggerblockadezeit gewählt. Die Mindestexspirationszeit kann auch so lange andauern, bis ein den Atemfluß oder das Atemzugvolumen repräsentierenden Signals anzeigt, daß der Exspirationsvorgang des Patienten abgeschlossen ist.

Das erfindungsgemäße Beatmungsgerät umfasst eine Atemgasquelle zum Erzeugen eines Gasdrucks für einen Patienten, der mittels einer Atemmaske abgeben wird, eine Messeinheit zum Messen einer für die Einatmung oder Ausatmung charakteristischen Messgröße und eine Steuereinheit, die aus der Messgröße das Tidalvolumen des Patienten bestimmt und mit einem Sollwert des Tidalvolumens vergleicht und die Atemgasquelle derart regelt, dass der erzeugte Gasdruck zur Verfügung gestellt wird bis der Sollwert des Tidalvolumens erreicht ist, wobei zusätzlich eine weitere Regelung zumindest einer für die Beatmung des Patienten charakteristischen Beatmungskenngröße erfolgt und ein entsprechendes Regelsignal an die Atemgasquelle abgegeben wird. Hierzu weist das Beatmungsgerät eine Steuereinheit auf, der die Atemgasquelle veranlasst, ausgehend vom einem Basis-Druckniveau, zumindest ein erhöhtes Druckniveau bereitzustellen wobei das Beatmungsgerät zudem eine Zähleinrichtung aufweist die mit dem Übergang auf das maximale Druckniveau aktiviert wird eine vorgebbare Zeitdauer zu zählen, wonach die Atemgasquelle vom Controller zur Absenkung des Atemgasdruckes veranlasst wird und wobei die Steuereinheit ferner ausgebildet ist, eine Ausgabeeinrichtung zur Ausgabe eines wahrnehmbaren Signals zu veranlassen.

Die erfindungsgemäße Vorrichtung unterstützt damit Patienten beim Abhusten dadurch, dass ausgehend vom Einatmungsdruck (IPAP) ein erhöhter Atemgasdruck bereitgestellt wird, wodurch eine gegenüber der normalen Beatmung tiefere Einatmung bewirkt und Lunge und Thorax vorgespannt werden. Für die Durchführung des Hustenstoßes ist patientenseitig eine gute Koordination mit einem geräteseitigen Druckmanöver notwendig. Hierzu muss der Patient im richtigen Moment vor dem Wiederabfall des Druckes seine Stimmritze (Glottis) schließen, um für einen Moment die Luft in der Lunge zu halten.

Durch ein vorrichtungsseitig generiertes Signal, welches bei Erreichen eines Druckplateaus bei einem erhöhten Atemgasdruck ertönt, kann der Patient den besten Zeitpunkt für den Schluss der Glottis bestimmen und sich optimal mit dem Gerät synchronisieren sowie im Anschluss selbst aktiv einen Hustenstoß durchführen. Gerätetechnisch wird ein optimaler Husten durch das schnelle Absenken des Druckes im Anschluss an die Insufflation unterstützt.

Die erfindungsgemäße Vorrichtung zeichnet sich vorteilhaft dadurch aus, dass die vorgebbare Zeitdauer im Bereich 0,250 bis 6 Sekunden ist. Diese Zeitdauer bietet dem Patienten ausreichend Zeit, einen Hustenstoß durch Schließen der Glottis vorzubereiten.

Für die erfindungsgemäße Steuereinheit zur Steuerung einer Atemgasquelle zur Beatmung eines Patienten umfassend eine Regelung des Tidalvolumens des Patienten durch Bereitstellen und Applizieren eines inspiratorischen Drucks und eine gleichzeitige Regelung einer weiteren Beatmungskenngröße gelten ebenfalls alle oben genannten Alternativen für die ergänzende Regelung einer (oder mehrerer) weiteren Beatmungskenngröße entsprechend. Hierbei ist unter der gleichzeitigen Regelung die Regelung zumindest zweier Beatmungskenngröße in einer Zeiteinheit gemeint oder die die Regelung zumindest zweier Beatmungskenngröße unter Berücksichtigung der jeweiligen Quereinflüsse oder Abhängigkeiten.

Erfindungsgemäß ist auch optional ein Pause-Modus vorgesehen. Eine Pause muss hier nicht bedeuten, dass die Maske währenddessen abgezogen wird. Es ist auch möglich, dass der Patient die Therapie fortsetzt, aber die Pause nutzt, um sich von hohen Therapiedrücken zu entlasten oder langsam wieder an eine Spontanatmung zu gewöhnen. Bevorzugt löst der Patient den Pause-Modus selbst aus. Nach Aktivierung des Pause-Modus durch den Patienten senkt das Beatmungsgerät die Beatmung schrittweise oder kontinuierlich ab. Hierbei ist auch ein zwischenzeitliches Wiederansteigen der Beatmung möglich, jedoch wird im Ergebnis die Beatmung im Pause-Modus zurückgefahren. Dies erfolgt durch Absenkung des IPAP und/oder EPAP und/oder der Frequenz und/oder des Volumens.

Die langsame Absenkung kann linear erfolgen: sprich eine feste Absenkung mindestens eines Druckes in hPa pro Atemzug oder pro Zeiteinheit, bevorzugt zwischen 0,1 und 10 hPa pro Minute.

Die langsame Absenkung kann nicht-linear erfolgen: sprich z. B. eine Absenkung mindestens eines Druckes in % pro Atemzug oder pro Zeiteinheit, bevorzugt zwischen 0,5 und 50 % pro Minute.

Die langsame Absenkung kann erfolgsbasiert erfolgen: nach einer einmaligen Absenkung in % oder hPa muss ein Erfolgs-Ereignis eintreten, um den nächsten Absenkungsschritt auszuführen. Erfolgs-Ereignisse können sein: Tastendruck / Spracheingabe / anderes Trigger-Ereignis durch Anwender, Erreichen oder Halten eines Zielwertes nach einer Zielzeit, z. B. kein Einbruch bei Atemvolumen oder SpO2 oder Atemfrequenz nach einer Messdauer zwischen 10s und 5min bzw. kein Anstieg des CO2 in dieser Zeit.

Für alle Ausführungsformen der Erfindung gilt zudem, dass bei der Regelung zumindest zweier Beatmungskenngrößen eine Priorisierung durch den Anwender oder automatisch vorgenommen werden kann, um ein Beatmungskenngröße stärker zu gewichten.

Die Erfindung wird nachfolgend anhand von Figuren erläutert. Es zeigen:
Fig. 1 den zeitlichen Verlauf des Tidalvolumens, Minutenvolumens und der Frequenz bei einem Patienten in einer stabilen Situation, wenn eine Beatmung mit einer vorgegebenen Druckdifferenz stattfindet, sowie den zeitlichen Verlauf des inspiratorischen Drucks, des exspiratorischen Drucks und der Druckdifferenz;
Fig. 2 die zeitlichen Verläufe aus Fig. 1 bei einer aktivierten Tidalvolumenregelung;
Fig. 3 die zeitlichen Verläufe bei einer aktivierten Tidalvolumenregelung in Kombination mit einer Auto-EPAP-Regelung;
Fig. 4 die zeitlichen Verläufe der oben genannten Kenngrößen bei einer aktivierten Tidalvolumenregelung und einer automatischen Frequenzregelung zur Steuerung des Minutenvolumenstroms;
Fig. 5a-c Ablaufdiagramme für die Kombination aus Tidalvolumenregelung und automatischer Frequenzregelung zur Steuerung des Minutenvolumens in drei Ausführungen; und
Fig. 6 die zeitlichen Verläufe bei einer aktivierten Tidalvolumenregelung zusätzlich zu einer antizyklischen Servoventilationsregelung.
Fig. 7 Ansicht eines Beatmungsgerätes mit einer Anzeigeeinheit und Schnittstellen

In der Figurenbeschreibung wird (abweichend von obigen Text) als Tidalvolumen stets das Atemvolumen pro Atemzug bezeichnet, während das Atemminutenvolumen oder Minutenvolumen das Tidalvolumen pro Minute beschreibt.

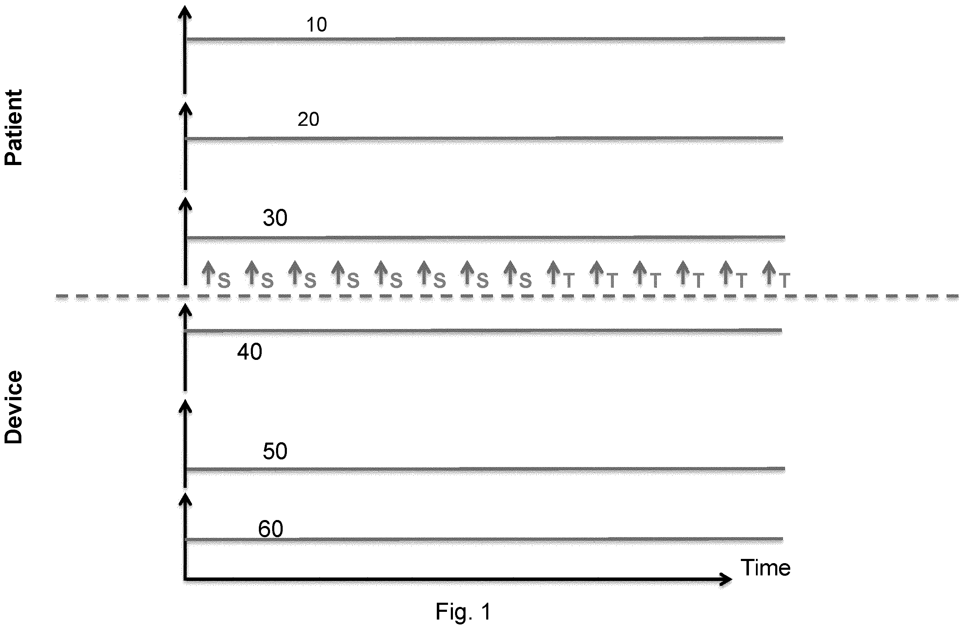

Fig. 1 zeigt im oberen Bereich drei zeitliche Verläufe bei einer stabilen Titration des Patienten über ein Beatmungsgerät 70. Gezeigt sind das Tidalvolumen 10 pro Atemzug, das Atemminutenvolumen 20 sowie die Atemfrequenz 30 des Patienten. Zusätzlich ist am unteren Rand des oberen Teils dargestellt, ob eine spontane Atmung durch den Patienten erfolgt (↑S) oder eine zeitlich gesteuerte Beatmung (TT), die durch das Beatmungsgerät 70 initiiert ist. Dabei wird entsprechend eine Hintergrundfrequenz von dem Gerät 70 vorgegeben und bei der Regelung eingehalten.

Im unteren Teil der Fig. 1 ist der zeitliche Verlauf des inspiratorischen Drucks 40 (IPAP), des exspiratorischen Drucks 50 (EPAP) sowie der Druckdifferenz 60 dargestellt. Da alle Kennlinien als horizontale Gerade dargestellt sind, erfolgt eine konstante zusätzliche Beatmung des Patienten, was zu einem konstanten Atemvolumen 10 und Atemminutenvolumen 20 führt.

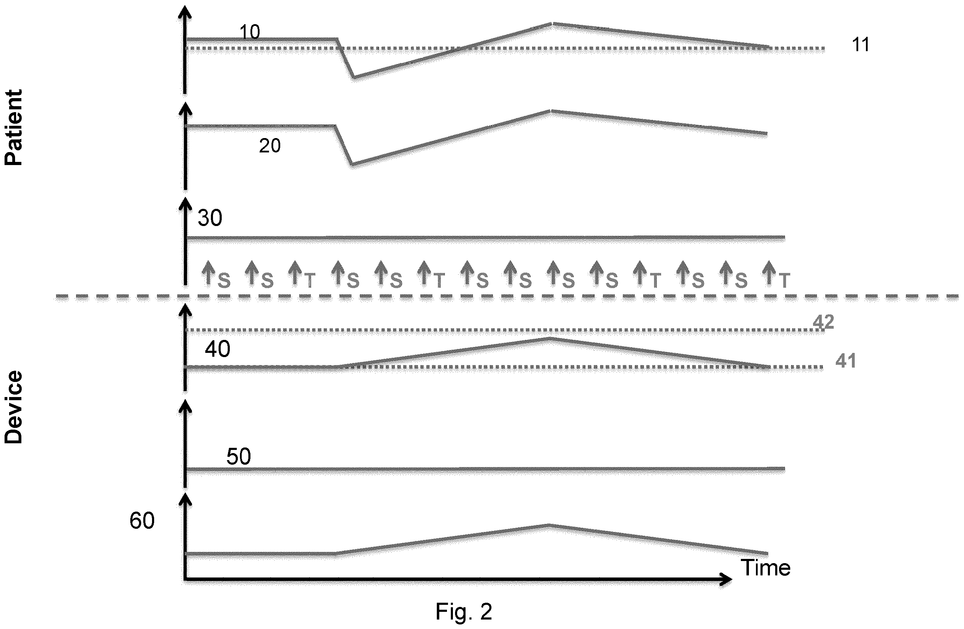

Fig. 2 zeigt hingegen den zeitlichen Verlauf bei einer aktivierten Tidalvolumenregelung. Fällt das Tidalvolumen 10 und das Minutenvolumen 20 des Patienten ab, so wird der Abfall von dem Beatmungsgerät 70 registriert und der inspiratorische Druck 40 erhöht. Der exspiratorische Druck 50 zur Öffnung der Atemwege bleibt konstant. Daraus ergibt sich eine Erhöhung der Druckdifferenz 60. Sobald der inspiratorische Druck 40 ansteigt, steigt auch das Tidalvolumen 10 des Patienten an. Wenn es den Sollwert 11 des Tidalvolumens überschreitet, beginnt die Regelung, beispielsweise nach einer gewissen Totzeit, erneut. Der inspiratorische Druck 40 wird erniedrigt, bis er wieder auf seinen Ausgangswert (untere Grenze 41) fällt. Der inspiratorische Druck 40 wird dabei innerhalb der vorgegebenen Druckgrenzen (IPAPmax als Obergrenze 42 und IPAPmin als Untergrenze 41) geregelt. In Fig. 2 ist zu erkennen, dass bei der hier gezeigten Regelung die maximale Obergrenze 42 des inspiratorischen Drucks 40 noch nicht erreicht wurde. EPAP 50 und/oder PEEP werden zudem oder ergänzend so geregelt, dass Obstruktionen weitgehend vermieden werden. Die Tidalvolumenregelung wird ergänzend dadurch verbessert, dass die Körpergröße des Patienten eingegeben wird, um daraus die anatomischen Toträume zu berechnen und bei der Beatmung zu berücksichtigen. Zusätzlich können weitere Patientendaten wie Alter oder Gewicht oder Geschlecht eingegeben werden. Unter Berücksichtigung der Patientendaten wird ein Zielwert für die alveoläre Ventilation errechnet und der EPAP 50 und/oder IPAP-Druck 40 so gesteuert, dass der Zielwert idealerweise erreicht wird.

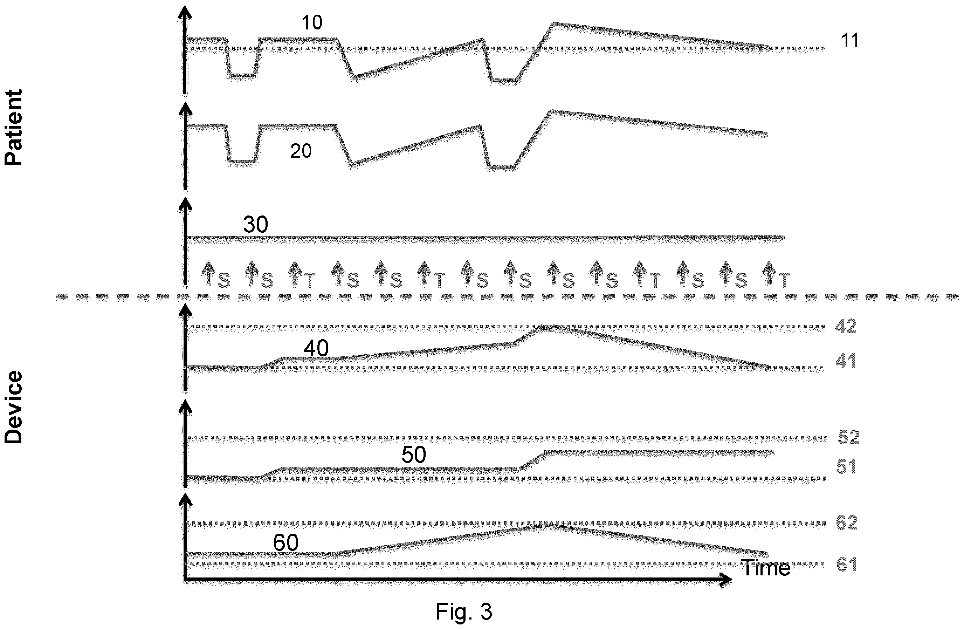

Fig. 3 zeigt die Kombination aus Tidalvolumenregelung und Auto-EPAP-Regelung. Im oberen Schaubild von Fig. 3 ist zu erkennen, dass das Tidalvolumen 10 und das Minutenvolumen 20 des Patienten zunächst sprungartig sinken. Dies kann beispielsweise durch zusätzliche Obstruktionen der oberen Luftwege erfolgen, beispielsweise bei einer Apnoe. Nach einem gewissen Zeitraum erhöht sich das Tidalvolumen 10 wieder selbständig und nimmt den vorherigen Wert erneut an, der in diesem Fall oberhalb eines Solltidalvolumens 11 (gestrichelte Linie) liegt.

Die Regelung reagiert auch hier mit einer zeitlichen Verzögerung. Der inspiratorische Druck 40 wird erhöht, um eine derartige Apnoe nochmals zu verhindern. Gleichzeitig wird in gleichem Maße der exspiratorische Druck 50 erhöht. Auch dieser Druck 50 kann innerhalb von vorgegebenen minimalen und maximalen Werten erfolgen, also zwischen einer gesetzten Untergrenze 51 und einer gesetzten Obergrenze 52. Da sowohl inspiratorischer Druck 40 wie auch exspiratorischer Druck 50 (gleichermaßen) erhöht wurden, bleibt die Druckdifferenz 60 zunächst konstant.

Im weiteren zeitlichen Verlauf erfolgt eine zweite Apnoe, bei der das Tidalvolumen 10 und das Minutenvolumen 20 des Patienten sinken. Nun erfolgt eine weitere Erhöhung des inspiratorischen Drucks 40, wobei der exspiratorische Druck 50 konstant bleibt. Konsequenterweise steigt auch die Druckdifferenz 60 an. Durch diese Regelung und das Verabreichen einer zeitlich gesteuerten Beatmung steigen das Tidalvolumen 10 und das Minutenvolumen 20 wieder an. Es erreicht wieder den Sollwert 11 des Tidalvolumens. Anschließend erfolgt eine weitere Reduktion des Tidalvolumens 10, beispielsweise durch eine erneute Apnoe. Hieraufhin wird der inspiratorische Druck 40 erhöht, bis der maximale Grenzwert 42 erreicht ist. Außerdem erfolgt eine Anhebung des exspiratorischen Drucks 50, um den Obstruktionen entgegenzuwirken. Durch diese Maßnahmen steigt in der Konsequenz das Tidalvolumen 10 wieder an, bis über den gewünschten Soll-Tidalvolumenwert 11. Der inspiratorische Druck 40 wird daraufhin reduziert, wobei der exspiratorische Druck 50 auf einem höheren Niveau, aber noch innerhalb der Druckgrenzen, verbleibt. Auf diese Weise lässt sich ein schnelles Einregeln des Tidalvolumens 10 des Patienten ermöglichen.

In den Fig. 1 bis 3 ist zu erkennen, dass die Frequenzregelung, und somit die Frequenz mit der das Beatmungsgerät 70 eine mandatorische Beatmung des Patienten durchführt, unabhängig von der Druckregulierung ist. Die Steuerung des Drucks erfolgt somit unabhängig von einer eingestellten Frequenzregulation; dies kann eine fest vorgegebene Frequenz sein oder eine eingestellte Hintergrundfrequenz, die zu festen Zeiten eine Beatmung des Patienten durchführt. Erfindungsgemäß ist auch vorgesehen, dass die Frequenz der Patientenatmung sensorisch 86 ermittelt wird und zumindest teilweise für die Berechnung der vorgegebenen Frequenz verwendet wird. Andere Messgrößen die in die Berechnung der vorgegebenen Frequenz einfließen können das Atemminutenvolumen und/oder das Zielvolumen sein.

Die vorgegebene Frequenz wird so berechnet und eingestellt, dass sie adaptiv hinter der Frequenz eines spontan atmenden Patienten zurückbleibt, sofern die spontane Patientenatmung innerhalb eines vorgebaren oder vorgegebenen Grenzwertes vom gewünschten Soll-Tidalvolumenwert 11 liegt. Sobald die spontane Patientenatmung außerhalb eines vorgebaren oder vorgegebenen Grenzwertes vom gewünschten Soll-Tidalvolumenwert 11 liegt, wird die vorgegebene Frequenz so berechnet und eingestellt, dass der gewünschte Soll-Tidalvolumenwert 11 erreicht wird oder die spontane Patientenatmung zumindest wieder innerhalb eines vorgebaren oder vorgegebenen Grenzwertes vom gewünschten Soll-Tidalvolumenwert 11 liegt.

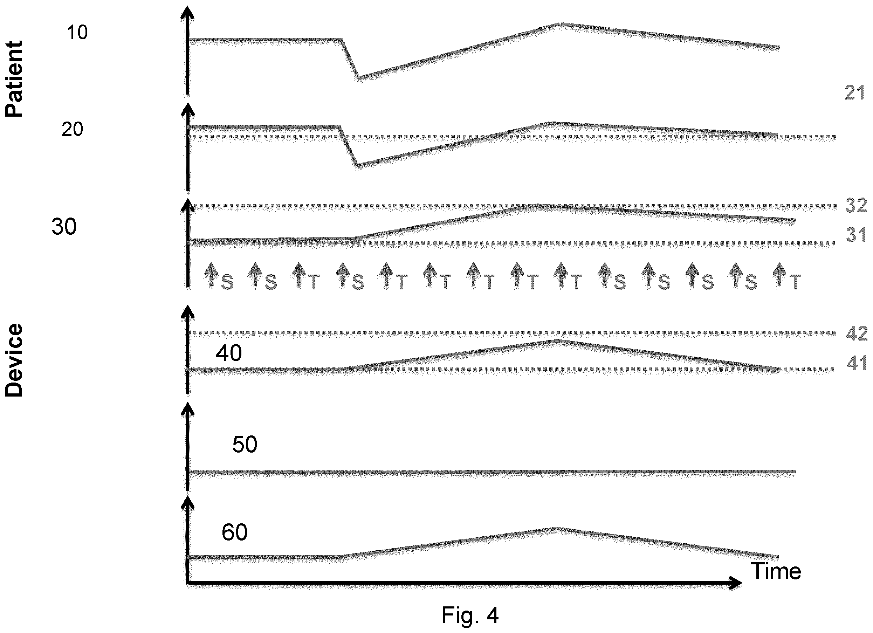

Fig. 4 zeigt die Kombination der Tidalvolumenregelung mit einer automatischen Frequenzregelung, um das Minutenvolumen 20 des Patienten anzugleichen. Erfolgt eine Absenkung des Tidalvolumens 10 und somit des Minutenvolumens 20 des Patienten, wird die Beatmungsfrequenz 30 erhöht. Bevorzugt erfolgt eine Frequenzregelung, mit der eine Beatmung erfolgt, innerhalb eines oberen 32 und unteren 31 Grenzwertes. Gleichzeitig mit der Anpassung und Erhöhung der Beatmungsfrequenz 30 wird auch der inspiratorische Druck 40 erhöht. Da der exspiratorische Druck 50 konstant bleibt, verläuft die Druckdifferenz 60 parallel zum inspiratorischen Druck 40. Zu erkennen ist, dass das Gerät die Reduktion des Atemvolumens 10 teilweise durch eine höhere Frequenz 30 der mandatorischen Beatmung kompensiert. Diese ist jedoch gepaart mit einer Anhebung des inspiratorischen Druckvolumens 40. Sobald das gewünschte Minutenvolumen 20 erzielt wird, also oberhalb eines Zielminutenvolumens 21 liegt, wird (nach einer gewissen Regeltotzeit) die Frequenz 30 wieder reduziert. Dabei erfolgt eine langsame Verkleinerung der Frequenz 30. Zusätzlich erfolgt mit einer weiteren Verzögerung auch eine Reduktion des inspiratorischen Drucks 40 bis dieser die vorgegebene Untergrenze 41 erreicht.

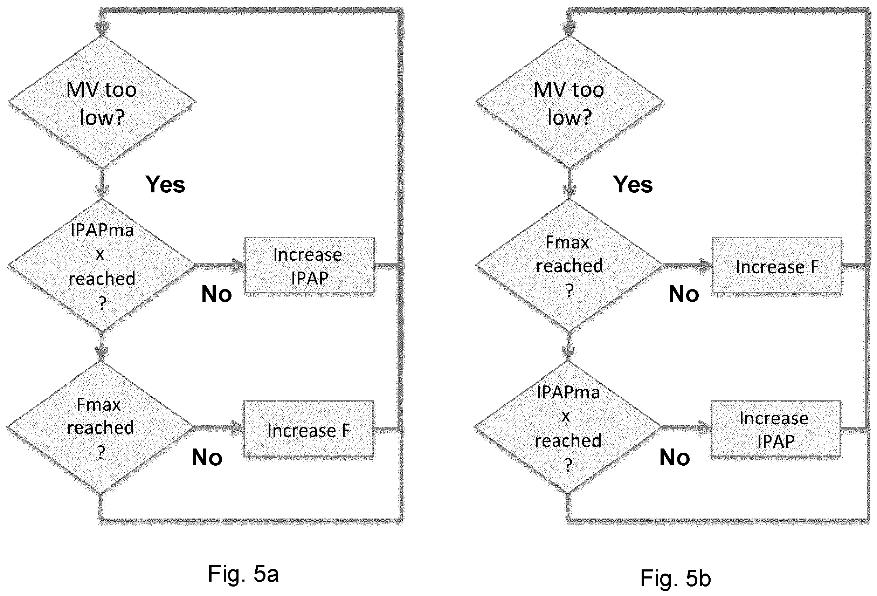

Die Fig. 5a - c zeigen je ein Ablaufdiagramm für die automatische Frequenzsteuerung gepaart mit der Tidalvolumenregelung. In Fig. 5a ist der Ablauf gezeigt, wenn der Regelschwerpunkt auf einem Anstieg des inspiratorischen Drucks liegt, Fig. 5b zeigt eine Regelung mit Schwerpunkt Frequenzregelung. Sobald das Minutenvolumen des Atemvolumens zu gering ist, wird zunächst überprüft, ob die Obergrenze des inspiratorischen Drucks (Fig. 5a) oder der Frequenz (Fig. 5b) erreicht ist. Ist dies nicht der Fall, erfolgt eine Anhebung des Drucks bzw. der Frequenz. Anschließend wird erneut überprüft, ob das Minutenvolumen zu gering ist.

Ist der erlaubte Grenzwert bereits erreicht, wird überprüft, ob das Maximum der entsprechenden anderen Regelgröße (Frequenz gemäß Fig. 5a bzw. Druck gemäß Fig. 5b) bereits erreicht ist. Ist dies nicht der Fall, erfolgt eine Erhöhung der Frequenz bzw. des Drucks; andernfalls nicht, da der Grenzwert nicht überschritten werden darf. Eine Regelung zur Erzeugung des Soll-Tidalvolumens ist dann nicht möglich.

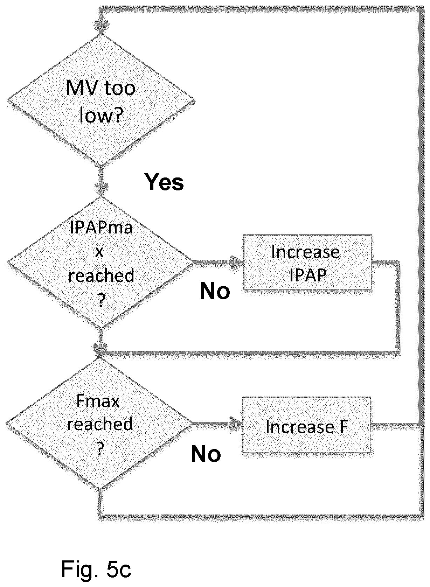

Fig. 5c zeigt eine Kombination aus beiden Regelungen. Auch hier wird wieder überprüft, ob das Minutenvolumen zu gering ist, also unter dem vorgegebenen Grenzwert liegt. Ist dies der Fall, erfolgt die Abfrage, ob der verabreichte inspiratorische Druck die Obergrenze bereits erreicht hat und unabhängig vom Ergebnis dieser Abfrage, ob die maximale Frequenz bereits appliziert ist. Ist eine Obergrenze noch nicht erreicht, erfolgt eine Erhöhung der jeweiligen Regelgröße, so dass sowohl der Druck als auch die Beatmungsfrequenz geregelt werden. Hierdurch lässt sich die Regelung beschleunigen, was schließlich zu einer Erhöhung des Komforts des Patienten führt.

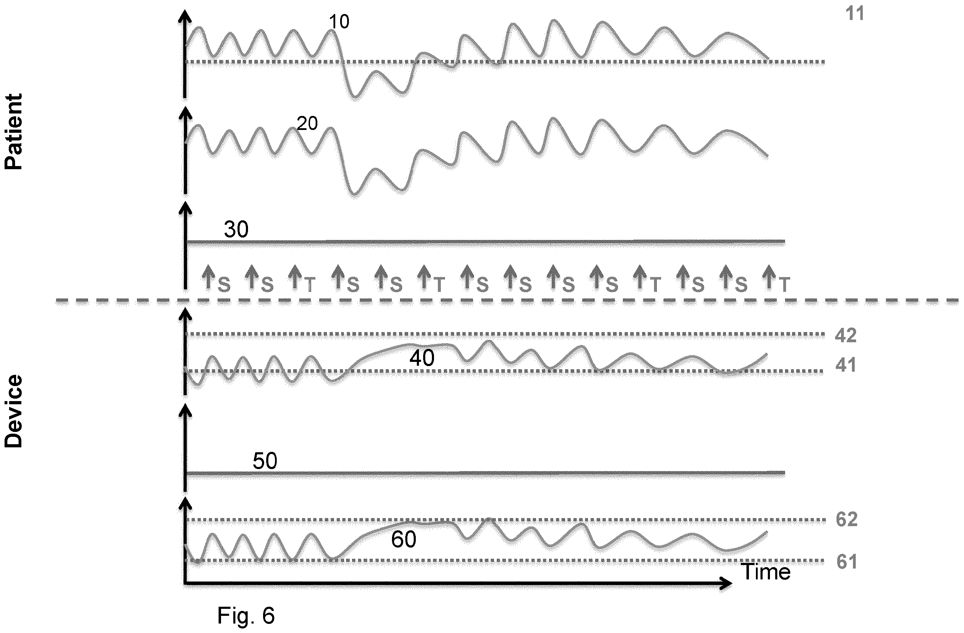

Fig. 6 zeigt den zeitlichen Verlauf einer Kombination aus Tidalvolumenregelung und antizyklischer Servoventilationsregelung. Die zeitlichen Verläufe des Tidalvolumens 10 und Minutenvolumens 20 zeigen ein typisches periodisches Atmen des Patienten bei einem sogenannten Cheyne-Stokes-Syndrom, bei der das Atemvolumen 10 um einen bestimmten Volumenwert schwankt. Um diese Schwankungen auszugleichen wird der inspiratorische Druck 40 antizyklisch angepasst. Bei einer Erhöhung des Tidalvolumens 10 wird der inspiratorische Druck 40 verringert; bei einer Verringerung des Tidalvolumens 10 der inspiratorische Druck 40 erhöht. Der exspiratorische Druck 50 bleibt konstant, so dass die Druckdifferenz 60 parallel zum inspiratorischen Druck 40 verläuft. Inspiratorischer Druck 40 und Druckdifferenz 60 werden innerhalb der vorgegebenen Grenzen (41, 42 bzw. 61, 62) geregelt.

Solange die periodische Atmung des Patienten oberhalb eines vorgegebenen Soll-Tidalvolumens 11 liegt, erfolgt auch die Regelung des inspiratorischen Drucks 40 um den unteren Grenzwert 41 herum. Er kann hierbei sogar unterschritten werden, da der untere Grenzwert 41 zur Einhaltung des Soll-Tidalvolumens 11 festgelegt ist. Kommt es zu einem Unterschreiten des Soll-Tidalvolumens 11, wird der inspiratorische Druck 40 erhöht. Die antizyklische Druckregelung wird aufgegeben und ein starker Anstieg des inspiratorischen Drucks 40 angelegt. Hierdurch wird die Reduktion des Luftvolumens 10 (Tidalvolumen) kompensiert. Sobald das Tidalvolumen 10 des Patienten den Soll-Tidalvolumenwert 11 wieder dauerhaft überschreitet, geht die Druckregelung wieder in eine antizyklische Regelung über. Allerdings ist das Druckniveau mit einem Offset versehen, der langsam sinkt, so dass der inspiratorische Druck 40 um den unteren Grenzwert 41 schwingt. Die antizyklische Druckregelung erfolgt oberhalb der Untergrenze 41 (IPAPmin).

Auf diese Weise lassen sich nicht nur die periodischen Volumenschwankungen des Patienten kompensieren, sondern auch schnell auf eine Reduktion des Atemvolumens reagieren.

Erfindungsgemäß ist auch eine Ermittlung von Leckagen vorgesehen. Leckagen sind zu berücksichtigen, da nur bei einer dichten Maske 75 die Therapiedrücke voll wirksam sind und Zielvolumina erreicht werden können. Sofern durch Sensoren 86 ein Anstieg der Leckage erkannt wird, erfolgt eine Ausgabe in Form einer Rückmeldung über die Maskenundichtigkeit an den Patient während der Therapie. Diese Ausgabe erfolgt bevorzugt am Display 71 des Gerätes 70.

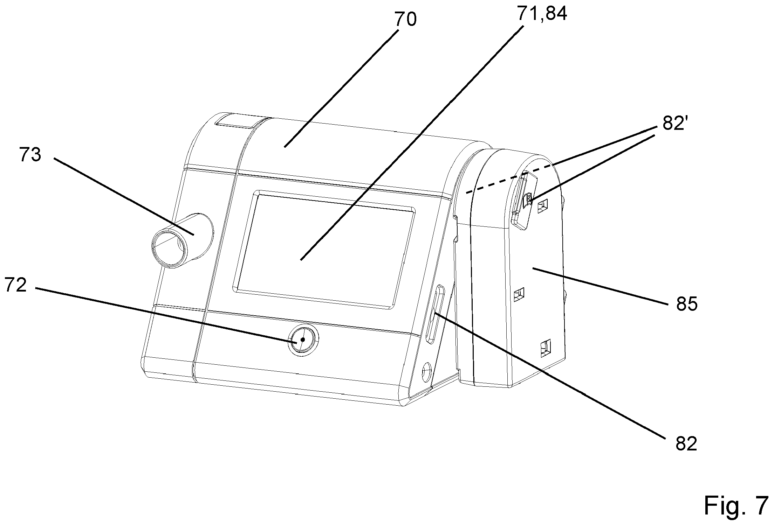

Fig.7 zeigt den grundsätzlichen Aufbau eines Beatmungsgerätes 70, welches mit einer Anzeigeeinrichtung 71, mindestens einem Bedienelement 72 sowie einem Schlauchanschluss 73 versehen ist. An den Schlauchanschluss 73 wird typischerweise über einen Verbindungsschlauch 74 eine Atemmaske 75 angeschlossen. Die Atemmaske 75 und der Verbindungsschlauch 74 zur Atemgasversorgung eines Patienten sind nicht dargestellt. Das Beatmungsgerät 70 weist eine innenliegende Atemgasquelle 76 in Form eines Gebläses sowie eine Steuerung 77 dieser Atemgasquelle 76 auf. Zusätzlich weist das Beatmungsgerät 70 auch eine Messeinheit 78 sowie eine Speichereinheit 80 auf. Die Anzeigeeinrichtung 71 dient neben der Anzeige auch als Bedieneinheit 84 einer Mensch-Maschine-Schnittstelle in Form eines Touchscreen-Displays. Das Beatmungsgerät 70 ist typischerweise mit mindestens einer Kommunikationsschnittstelle 82 versehen. Die Schnittstelle 82 ist hier in einem Seitenbereich angeordnet. Die Schnittstelle 82 ist zur Horizontalen geneigt und befindet sich in räumlicher Nähe zur Anzeigeeinrichtung 71. Bevorzugt weist das Beatmungsgerät 70 zudem eine weitere Schnittstelle 82' auf, die der Kopplung von Modulen 85 dient.
- Verfahren zur Steuerung einer Atemgasquelle (76) mit einer Steuereinheit (77) zur Beatmung eines Patienten umfassend eine Regelung des Tidalvolumens (10) des Patienten durch Bereitstellen und Applizieren eines inspiratorischen Drucks (40) und eine gleichzeitige Regelung einer weiteren Beatmungskenngröße.
- Das Verfahren ist auch dadurch gekennzeichnet, dass die Regelung des Tidalvolumens (10) die folgenden Schritte umfasst:
   - messen des Gasstroms, des Volumenstroms oder einer äquivalenten Atemgasgröße
   - Bestimmen des von dem Patienten empfangenen Luftvolumens
   - Festlegen eines Sollwertes (11) des Tidalvolumens des Patienten
   - Erzeugen eines inspiratorischen Drucks (40)
   - Regeln des inspiratorischen Drucks (40) derart, dass der Sollwert (11) des Tidalvolumens erreicht wird.
- Das Verfahren ist auch dadurch gekennzeichnet, dass die Regelung der Beatmungskenngröße eine der folgenden Regelungen ist:
   - Regelung über einen automatischen Trigger zur Erkennung einer Ausatmung und/oder einer Einatmung,
   - automatische Anpassung des Ausatemdrucks,
   - automatische Regelung der Beatmungsfrequenz (30),
   - antizyklische Druckreaktions-Regelung,
   - Regelung des Übergangdrucks zwischen inspiratorischem Druck (40) und exspiratorischem Druck (50).
- Das Verfahren ist auch dadurch gekennzeichnet, dass ein exspiratorischer Druck (50) erzeugt wird, der geringer als der inspiratorische Druck (40) ist, so dass eine Druckdifferenz (60) gebildet wird, wobei bevorzugt der inspiratorische Druck (40) zwischen einer unteren inspiratorischen Druckgrenze (41) und einer oberen inspiratorischen Druckgrenze (42) veränderbar ist und bevorzugt der exspiratorische Druck (50) zwischen einer unteren exspiratorischen Druckgrenze (51) und einer oberen exspiratorischen Druckgrenze (52) veränderbar ist.
- Das Verfahren ist auch dadurch gekennzeichnet, dass die Druckdifferenz (60) geregelt wird, bis das von dem Patienten empfangene Tidalvolumen (10) gleich dem Sollwert (11) des Tidalvolumens ist.
- Das Verfahren ist auch dadurch gekennzeichnet, dass die Regelung des Tidalvolumens (10) über einen oder mehrere Atemzüge des Patienten erfolgt, wobei die mandatorische Beatmung auf eine vorhandene Spontanatmung des Patienten bevorzugt abgestimmt ist.
- Das Verfahren ist auch dadurch gekennzeichnet, dass eine Einatmung und eine Ausatmung von der Steuereinheit (77) durch zyklische Messung und Auswertung eines Atemparameters erkannt und das momentane Tidalvolumen (10) gemessen und mit dem Sollwert (11) des Tidalvolumens verglichen wird und in einem weiteren Verfahrensschritt bei Unterschreitung des Sollwertes (11) der inspiratorische Druck (40) oder die Druckdifferenz (60) zwischen dem inspiratorischen Druck (40) und dem exspiratorischen Druck (50) erhöht wird.
- Das Verfahren ist auch dadurch gekennzeichnet, dass der exspiratorische Druck (50) automatisch anhand eines vorgegebenen Therapiezieles angepasst wird.
- Das Verfahren ist auch dadurch gekennzeichnet, dass bei zusätzlicher Regelung der Beatmungsfrequenz (30) und einer mandatorischen Beatmung eine automatische Regelung der Beatmungsfrequenz (30) in Abhängigkeit von der Spontanfrequenz des Patienten erfolgt, wobei sie bevorzugt als fester Prozentsatz der Spontanatemfrequenz, als feste Differenz zur Spontanatemfrequenz oder iterativ veränderbar ist, um eine beschleunigte oder verlangsamte Spontanatmung zu erzeugen.
- Das Verfahren ist auch dadurch gekennzeichnet, dass der Sollwert (11) des Tidalvolumens als ein unterer Grenzwert hinterlegt ist und bei einem Tidalvolumen (10) oberhalb des Grenzwertes (11) eine zusätzliche antizyklische Druckreaktion erfolgt, um einer Schwankung des Tidalvolumens (10) entgegenzuwirken.
- Das Verfahren ist auch dadurch gekennzeichnet, dass die applizierte Druckdifferenz (60) zwischen dem inspiratorischen Druck (40) und dem exspiratorischen Druck (50) verändert wird in Abhängigkeit der Volumendifferenz zwischen aktuell gemessenem Tidalvolumen (10) und dem Sollwert (11) des Tidalvolumens, wobei bevorzugt eine adaptive Anpassung erfolgt, besonders bevorzugt mit einer vorgegebenen Rampenzeit, nach der der Sollwert erreicht ist und die Druckdifferenz ihr Maximum erreicht hat, oder mit einem vorgegebenen Anteil von Inspiration und/oder Exspiration oder über eine Regelung mit einem vorgegebenen Druckprofil.
- Das Verfahren ist auch dadurch gekennzeichnet, dass bei der Bestimmung des Tidalvolumens (10) das Totraumvolumen des Patienten berücksichtigt wird.
- Beatmungsgerät (70) umfassend eine Atemgasquelle (76) zum Erzeugen eines Gasdrucks für einen Patienten, der mittels einer Atemmaske (75) abgeben wird, eine Messeinheit (78) zum Messen einer für die Einatmung oder Ausatmung charakteristischen Messgröße und eine Steuereinheit (77), die aus der Messgröße das Tidalvolumen (10) des Patienten bestimmt und mit einem Sollwert (11) des Tidalvolumens vergleicht und die Atemgasquelle (76) derart regelt, dass der erzeugte Gasdruck zur Verfügung gestellt wird bis der Sollwert (11) des Tidalvolumens erreicht ist, wobei zusätzlich eine weitere Regelung einer für die Beatmung des Patienten charakteristischen Beatmungskenngröße erfolgt und ein entsprechendes Regelsignal an die Atemgasquelle (76) abgegeben wird.
- Das Beatmungsgerät (70) ist auch dadurch gekennzeichnet, dass die Steuereinheit (77) zur Ausführung der Verfahrensschritte nach einem der Ansprüche 1 bis 15 ausgebildet und eingerichtet ist.
- Das Beatmungsgerät (70) ist auch dadurch gekennzeichnet, dass es
   - eine Alarmierungseinheit (79) umfasst, um ein Alarmsignal auszugeben, bevorzugt ein Tonsignal, wobei die Alarmierungseinheit (79) bevorzugt ein Lautsprecher oder ein Buzzer ist,
   - eine Anzeigeeinheit (71), bevorzugt ein Display umfasst, um Parameter und/oder Beatmungskenngrößen und/oder Messwerte und/oder aus Messwerten weiter verarbeitete Werte anzuzeigen oder an ein externes Anzeigegerät (83) über eine Schnittstelle (82) für eine Anzeige zu übermitteln,
   - eine Speichereinheit (80) zur Abspeicherung der gemessenen Werte, der charakteristischen Beatmungskenngrößen, der Alarme, der Atemvolumen oder der Einstellparameter und eine Recheneinheit (81) zur statistischen Auswertung,
   - eine Bedieneinheit (84), die eine oder mehrere Mensch-Maschine-Schnittstellen aufweist und bevorzugt ein Touchscreen-Display (71) hat.

### Pos.-Nr.:

- 10: Tidalvolumen pro Atemzug
- 11: Sollwert des Tidalvolumen
- 20: Atemminutenvolumen
- 30: Atemfrequenz
- ↑S: spontane Atmung
- ↑T: gesteuerte Beatmung
- 40: inspiratorischer Druck, IPAP
- 41: IPAPmin, untere Grenze bzw. Ausgangswert
- 42: IPAPmax, Obergrenze IPAP
- 50: exspiratorischer Druck, EPAP
- 51: EPAPmin, Untergrenze EPAP
- 52: EPAPmax, Obergrenze EPEP
- 60: Druckdifferenz
- 61: Untergrenze Druckdifferenz
- 62: Obergrenze Druckdifferenz
- 70: Beatmungsgerät
- 71: Anzeigeeinrichtung, z.B. Display
- 72: Bedienelement
- 73: Schlauchanschluss
- 74: Verbindungsschlauch
- 75: Atemmaske
- 76: Atemgasquelle
- 77: Steuereinheit
- 78: Messeinheit
- 79: Alarmierungseinheit z. B. Lautsprecher, Buzzer
- 80: Speichereinheit
- 81: Recheneinheit
- 82: Schnittstelle
- 82': Schnittstelle
- 83: externes Anzeigegerät
- 84: Bedieneinheiten
- 85: Modul
- 86: Sensor

## Patentansprüche

1. Steuereinheit (77) zur Steuerung einer Atemgasquelle (76) zur Beatmung eines Patienten umfassend eine Regelung des Tidalvolumens (10) des Patienten durch Bereitstellen und Applizieren eines inspiratorischen Drucks (40) und eine gleichzeitige Regelung einer weiteren Beatmungskenngröße **dadurch gekennzeichnet, dass** als weitere Beatmungskenngröße neben der Änderung des Tidalvolumens auch eine Beatmungsfrequenz für eine mandatorische Beatmung geregelt wird.

2. Steuereinheit (77) nach Anspruch 1, **dadurch gekennzeichnet, dass** bei dieser Art der mandatorischen Beatmung eine automatische Regelung der Beatmungsfrequenz in Abhängigkeit von der Spontanfrequenz des Patienten, die von der Steuereinheit ermittelt wird, erfolgt.

3. Steuereinheit (77) nach Anspruch 1 oder 2, **dadurch gekennzeichnet, dass** der Abstand der mandatorischen Atemzüge zueinander und zum letzten spontanen Atemzug von der Steuereinheit (77) selbständig bestimmt.

4. Steuereinheit (77) nach zumindest einem der vorhergehenden Ansprüche, **dadurch gekennzeichnet, dass** die mandatorische Atemfrequenz als Prozentsatz der Spontanatemfrequenz oder als festes Delta zur Spontanatemfrequenz, also mit einer festen Differenz, bestimmt wird.

5. Steuereinheit (77) nach zumindest einem der vorhergehenden Ansprüche, **dadurch gekennzeichnet, dass** eine iterative Beschleunigung oder Verlangsamung der Frequenz erfolgt, mit dem Ziel der Erreichung einer höheren oder niedrigeren Quote an spontanen Atemzügen.

6. Steuereinheit (77) nach zumindest einem der vorhergehenden Ansprüche, **dadurch gekennzeichnet, dass** EPAP und/oder PEEP so gewählt werden, dass Obstruktionen der oberen Atemwege vermieden werden und zudem der Sollwert für das Tidalvolumen erreicht wird, wobei ebenfalls die Frequenz angepasst wird.

7. Steuereinheit (77) nach zumindest einem der vorhergehenden Ansprüche, **dadurch gekennzeichnet, dass** bei Absenkung des Tidalvolumens und somit des Minutenvolumens des Patienten, die Beatmungsfrequenz erhöht wird.

8. Steuereinheit (77) nach zumindest einem der vorhergehenden Ansprüche, **dadurch gekennzeichnet, dass** bei Anstieg des EPAP ebenfalls die Beatmungsfrequenz erhöht wird, um das Tidalvolumen zu erreichen.

9. Steuereinheit (77) nach zumindest einem der vorhergehenden Ansprüche, **dadurch gekennzeichnet, dass** die Frequenzregelung, und somit die Frequenz mit der das Beatmungsgerät (70) eine mandatorische Beatmung des Patienten durchführt, unabhängig von der Druckregulierung ist.

10. Steuereinheit (77) nach zumindest einem der vorhergehenden Ansprüche, **dadurch gekennzeichnet, dass** die Steuerung des Drucks unabhängig von einer eingestellten Frequenzregulation erfolgt, wobei diese eine fest vorgegebene Frequenz oder eine eingestellte Hintergrundfrequenz ist, die zu festen Zeiten eine Beatmung des Patienten durchführt.

11. Steuereinheit (77) nach zumindest einem der vorhergehenden Ansprüche, **dadurch gekennzeichnet, dass** die Frequenz der Patientenatmung sensorisch (86) ermittelt wird und zumindest teilweise für die Berechnung der vorgegebenen Frequenz verwendet wird.

12. Steuereinheit (77) nach zumindest einem der vorhergehenden Ansprüche, **dadurch gekennzeichnet, dass** andere Messgrößen in die Berechnung der vorgegebenen Frequenz einfließen nämlich das Atemminutenvolumen und/oder das Zielvolumen.

13. Steuereinheit (77) nach zumindest einem der vorhergehenden Ansprüche, **dadurch gekennzeichnet, dass** die vorgegebene Frequenz so berechnet und eingestellt wird, dass sie adaptiv hinter der Frequenz eines spontan atmenden Patienten zurückbleibt, sofern die spontane Patientenatmung innerhalb eines vorgebaren oder vorgegebenen Grenzwertes vom gewünschten Soll-Tidalvolumenwert (11) liegt.

14. Steuereinheit (77) nach zumindest einem der vorhergehenden Ansprüche, **dadurch gekennzeichnet, dass** sobald die spontane Patientenatmung außerhalb eines vorgebaren oder vorgegebenen Grenzwertes vom gewünschten Soll-Tidalvolumenwert (11) liegt, die vorgegebene Frequenz so berechnet und eingestellt wird, dass der gewünschte Soll-Tidalvolumenwert (11) erreicht wird oder die spontane Patientenatmung zumindest wieder innerhalb eines vorgebaren oder vorgegebenen Grenzwertes vom gewünschten Soll-Tidalvolumenwert (11) liegt.

15. Steuereinheit (77) nach zumindest einem der vorhergehenden Ansprüche, **dadurch gekennzeichnet, dass** die Kombination der Tidalvolumenregelung mit einer automatischen Frequenzregelung erfolgt, um das Minutenvolumen (20) des Patienten anzugleichen, wobei, wenneine Absenkung des Tidalvolumens (10) und somit des Minutenvolumens 20 des Patienten festgestellt wird, die Beatmungsfrequenz (30) erhöht wird.

16. Steuereinheit (77) nach zumindest einem der vorhergehenden Ansprüche, **dadurch gekennzeichnet, dass** gleichzeitig mit der Anpassung und Erhöhung der Beatmungsfrequenz (30) auch der inspiratorische Druck (40) erhöht wird.

17. Steuereinheit (77) nach zumindest einem der vorhergehenden Ansprüche, **dadurch gekennzeichnet, dass** eine Reduktion des Atemvolumens (10) durch eine höhere Frequenz 30 der mandatorischen Beatmung kompensiert wird.

18. Steuereinheit (77) nach zumindest einem der vorhergehenden Ansprüche, **dadurch gekennzeichnet, dass** sobald das gewünschte Minutenvolumen (20) erzielt wird, dieses also oberhalb eines Zielminutenvolumens (21) liegt, die Frequenz (30) wieder reduziert wird.

19. Steuereinheit (77) nach zumindest einem der vorhergehenden Ansprüche,, **dadurch gekennzeichnet, dass** bei zusätzlicher Regelung der Beatmungsfrequenz (30) und einer mandatorischen Beatmung eine automatische Regelung der Beatmungsfrequenz (30) in Abhängigkeit von der Spontanfrequenz des Patienten erfolgt, wobei sie bevorzugt als fester Prozentsatz der Spontanatemfrequenz, als feste Differenz zur Spontanatemfrequenz oder iterativ veränderbar ist, um eine beschleunigte oder verlangsamte Spontanatmung zu erzeugen.

20. Beatmungsgerät (70) umfassend eine Atemgasquelle (76) zum Erzeugen eines Gasdrucks für einen Patienten, der mittels einer Atemmaske (75) abgeben wird, eine Messeinheit (78) zum Messen einer für die Einatmung oder Ausatmung charakteristischen Messgröße und eine Steuereinheit (77), die aus der Messgröße das Tidalvolumen (10) des Patienten bestimmt und mit einem Sollwert (11) des Tidalvolumens vergleicht und die Atemgasquelle (76) derart regelt, dass der erzeugte Gasdruck zur Verfügung gestellt wird bis der Sollwert (11) des Tidalvolumens erreicht ist, wobei zusätzlich eine weitere Regelung einer für die Beatmung des Patienten charakteristischen Beatmungskenngröße erfolgt und ein entsprechendes Regelsignal an die Atemgasquelle (76) abgegeben wird **dadurch gekennzeichnet, dass** zur Ausführung der Schritte, die Steuereinheit (77) nach einem der Ansprüche 1 bis 19 ausgebildet und eingerichtet ist.

## Claims

1. A control unit (77) for controlling a respiration gas source (76) for ventilating a patient, comprising a regulation of the tidal volume (10) of the patient by providing and applying an inspiratory pressure (40), and a simultaneous regulation of another ventilation parameter, **characterized in that**, beyond the change in the tidal volume, a ventilation frequency for mandatory ventilation is also regulated as another ventilation parameter.

2. The control unit (77) according to claim 1, **characterized in that** with this type of mandatory ventilation, the ventilation frequency is automatically regulated depending on the spontaneous frequency of the patient which is ascertained by the control unit.

3. The control unit (77) according to claim 1 or 2, **characterized in that** the spacing of the mandatory breaths to each other and to the last spontaneous breath is determined independently by the control unit (77).

4. The control unit (77) according to at least one of the preceding claims, **characterized in that** the mandatory breathing rate is determined as a percentage of the spontaneous breathing rate, or as a fixed delta to the spontaneous breathing rate, that is, with a fixed difference.

5. The control unit (77) according to at least one of the preceding claims, **characterized in that** there is an iterative acceleration or deceleration of the frequency with the goal of achieving a higher or lower rate of spontaneous breaths.

6. The control unit (77) according to at least one of the preceding claims, **characterized in that** the EPAP and/or PEEP are selected such that obstructions of the upper airways are avoided, and moreover the setpoint for the tidal volume is achieved, wherein the frequency is also adjusted.

7. The control unit (77) according to at least one of the preceding claims, **characterized in that** upon lowering the tidal volume and therefore the minute volume of the patient, the ventilation frequency is increased.

8. The control unit (77) according to at least one of the preceding claims, **characterized in that** upon increasing the EPAP, the ventilation frequency is also increased in order to achieve the tidal volume.

9. The control unit (77) according to at least one of the preceding claims, **characterized in that** the frequency regulation and therefore the frequency with which the artificial respiration device (70) performs mandatory ventilation of the patient, is independent of the pressure regulation.

10. The control unit (77) according to at least one of the preceding claims, **characterized in that** the pressure is controlled independent of a set frequency regulation, wherein this is a fixed predetermined frequency or an adjusted background frequency which performs ventilation of the patient at fixed times.

11. The control unit (77) according to at least one of the preceding claims, **characterized in that** the frequency of patient breathing is determined by sensors (86) and is used at least partially for calculating the specified frequency.

12. The control unit (77) according to at least one of the preceding claims, **characterized in that** other measured quantities are incorporated in the calculation of the specified frequency, namely the respiratory minute volume and/or the target volume.

13. The control unit (77) according to at least one of the preceding claims, **characterized in that** the specified frequency is calculated and adjusted such that it adaptively remains behind the frequency of a spontaneously breathing patient, provided that the spontaneous patient breathing lies within a specified or specifiable limit value of the desired target tidal volume value (11).

14. The control unit (77) according to at least one of the preceding claims, **characterized in that** once the spontaneous patient respiration lies outside a specified or specifiable limit value of the desired target tidal volume value (11), the specified frequency is calculated and adjusted such that the desired target tidal volume value (11) is reached, or the spontaneous patient respiration at least again lies within a specified or specifiable limit value of the desired target tidal volume value (11).

15. The control unit (77) according to at least one of the preceding claims, **characterized in that** the combination of the tidal volume regulation is performed with an automatic frequency regulation in order to adjust the minute volume (20) of the patient, wherein, if a reduction of the tidal volume (10) and therefore of the minute volume (20) of the patient is detected, the ventilation frequency (30) is increased.

16. The control unit (77) according to at least one of the preceding claims, **characterized in that** the inspiratory pressure (40) is also increased at the same time as the adaptation and increase of the ventilation frequency (30).

17. The control unit (77) according to at least one of the preceding claims, **characterized in that** a reduction in the respiratory volume (10) is compensated for by a higher frequency (30) of mandatory ventilation.

18. The control unit (77) according to at least one of the preceding claims, **characterized in that** once the desired minute volume (20) is achieved, that is, it is above a target minute volume (21), the frequency (30) is again reduced.

19. The control unit (77) according to at least one of the preceding claims, **characterized in that**, with additional regulation of the ventilation frequency (30) and mandatory ventilation, the ventilation frequency (30) is automatically regulated depending on the spontaneous frequency of the patient, wherein it can be preferably changeable as a fixed percentage of the spontaneous breathing rate, as a fixed difference from the spontaneous breathing rate or iteratively in order to produce accelerated or decelerated spontaneous breathing.

20. An artificial respiration device (70) comprising a respiration gas source (76) for generating a gas pressure for a patient, which is delivered by means of a respiratory mask (75), a measuring unit (78) for measuring a measurement quantity typical of inhalation or exhalation and a control unit (77) which determines the patient's tidal volume (10) from the measurement quantity and compares this with a setpoint value (11) of the tidal volume and regulates the respiration gas source (76) such that the generated gas pressure is made available until the setpoint value (11) of the tidal volume is reached, wherein a further regulation of a ventilation characteristic variable which is typical of the patient's ventilation is additionally carried out and a corresponding regulating signal is delivered to the respiration gas source (76), **characterized in that** to perform the steps, the control unit (77) is designed and configured according to one of claims 1 to 19.

## Revendications

1. Unité de commande (77) pour la commande d'une source de gaz respiratoire (76) pour la ventilation d'un patient, comprenant une régulation du volume courant (10) du patient par la mise à disposition et l'application d'une pression inspiratoire (40) et une régulation simultanée d'un autre paramètre respiratoire, **caractérisée en ce que**, comme autre paramètre respiratoire, une fréquence respiratoire pour une ventilation imposée est également régulée en plus de la modification du volume courant.

2. Unité de commande (77) selon la revendication 1, **caractérisée en ce que**, sur ce type de ventilation imposée, une régulation automatique de la fréquence respiratoire est effectuée en fonction de la fréquence spontanée du patient qui est déterminée par l'unité de commande.

3. Unité de commande (77) selon la revendication 1 ou 2, **caractérisée en ce que** l'écart entre les différentes respirations imposées entre elles, et avec la dernière respiration spontanée, est déterminé de façon autonome par l'unité de commande (77).

4. Unité de commande (77) selon au moins l'une des revendications précédentes, **caractérisée en ce que** la fréquence respiratoire imposée est déterminée comme pourcentage de la fréquence respiratoire spontanée ou comme delta fixe par rapport à la fréquence respiratoire spontanée, donc avec une différence fixe.

5. Unité de commande (77) selon au moins l'une des revendications précédentes, **caractérisée en ce qu'**une accélération itérative ou un ralentissement itératif de la fréquence sont effectués dans le but d'obtenir un taux de respirations spontanées plus élevé ou plus bas.

6. Unité de commande (77) selon au moins l'une des revendications précédentes, **caractérisée en ce que** l'EPAP et/ou la PEP sont sélectionnées de telle sorte que les obstructions des voies respiratoires supérieures sont évitées et que, de plus, la valeur prescrite pour le volume courant est atteinte, la fréquence étant également ajustée.

7. Unité de commande (77) selon au moins l'une des revendications précédentes, **caractérisée en ce que** lors de la diminution du volume courant, et donc du volume minute du patient, la fréquence de ventilation est augmentée.

8. Unité de commande (77) selon au moins l'une des revendications précédentes, **caractérisée en ce que**, lors de l'augmentation de l'EPAP, la fréquence de ventilation est également augmentée afin d'atteindre le volume courant.

9. Unité de commande (77) selon au moins l'une des revendications précédentes, **caractérisée en ce que** la régulation de fréquence, et donc la fréquence avec laquelle l'appareil respiratoire (70) exécute une ventilation imposée du patient, est indépendante de la régulation de la pression.

10. Unité de commande (77) selon au moins l'une des revendications précédentes, **caractérisée en ce que** la commande de la pression est effectuée indépendamment d'une régulation de fréquence réglée, celle-ci étant une fréquence fixe spécifiée ou une fréquence d'arrière-plan réglée, laquelle exécute une ventilation du patient à des instants fixes.

11. Unité de commande (77) selon au moins l'une des revendications précédentes, **caractérisée en ce que** la fréquence de la respiration du patient est déterminée par capteur (86) et est utilisée au moins partiellement pour le calcul de la fréquence spécifiée.

12. Unité de commande (77) selon au moins l'une des revendications précédentes, **caractérisée en ce que** d'autres grandeurs de mesure sont intégrées au calcul de la fréquence spécifiée, à savoir le volume minute respiratoire et/ou le volume cible.

13. Unité de commande (77) selon au moins l'une des revendications précédentes, **caractérisée en ce que** la fréquence spécifiée est calculée et réglée de telle sorte qu'elle reste derrière la fréquence d'un patient respirant spontanément, de façon adaptative, dans la mesure où la respiration spontanée du patient se trouve en deçà d'une valeur limite spécifiable ou spécifiée de la valeur de volume courant prescrite souhaitée (11).

14. Unité de commande (77) selon au moins l'une des revendications précédentes, **caractérisée en ce que** la fréquence spécifiée est calculée et réglée de telle sorte que la valeur de volume courant prescrite souhaitée (11) est atteinte ou que la respiration spontanée du patient se trouve de nouveau au moins en deçà d'une valeur limite spécifiable ou spécifiée de la valeur de volume courant prescrite souhaitée (11), dès que la respiration spontanée du patient se trouve au-delà d'une valeur limite spécifiable ou spécifiée de la valeur de volume courant prescrite souhaitée (11).

15. Unité de commande (77) selon au moins l'une des revendications précédentes, **caractérisée en ce que** la combinaison de la régulation de volume courant est effectuée avec une régulation de fréquence automatique pour compenser le volume minute (20) du patient, la fréquence de ventilation (30) étant augmentée lorsqu'une diminution du volume courant (10), et donc du volume minute (20) du patient, est constatée.

16. Unité de commande (77) selon au moins l'une des revendications précédentes, **caractérisée en ce que** la pression inspiratoire (40) aussi est augmentée, simultanément à l'ajustement et à l'augmentation de la fréquence respiratoire (30).

17. Unité de commande (77) selon au moins l'une des revendications précédentes, **caractérisée en ce qu'**une réduction du volume respiratoire (10) est compensée par une fréquence (30) plus élevée de la ventilation imposée.

18. Unité de commande (77) selon au moins l'une des revendications précédentes, **caractérisée en ce que** la fréquence (30) est de nouveau réduite dès que le volume minute (20) souhaité est atteint, donc lorsque celui-ci est supérieur à un volume minute cible (21).

19. Unité de commande (77) selon au moins l'une des revendications précédentes, **caractérisée en ce qu'**une régulation automatique de la fréquence respiratoire (30) est effectuée en fonction de la fréquence spontanée du patient, lors d'une régulation supplémentaire de la fréquence respiratoire (30) et d'une ventilation imposée, laquelle étant modifiable de préférence comme un pourcentage fixe de la fréquence respiratoire spontanée, comme une différence fixe par rapport à la fréquence respiratoire spontanée ou de façon itérative, afin de produire une respiration spontanée accélérée ou ralentie.

20. Appareil respiratoire (70) comprenant une source de gaz respiratoire (76) pour générer une pression de gaz pour un patient, laquelle est délivrée au moyen d'un masque respiratoire (75), une unité de mesure (78) pour mesurer une grandeur de mesure caractéristique pour l'inspiration ou l'expiration et une unité de commande (77) qui détermine le volume courant (10) du patient à partir de la grandeur de mesure et le compare à une valeur prescrite (11) du volume courant et régule la source de gaz respiratoire (76) de telle sorte que la pression de gaz générée est mise à disposition jusqu'à ce que la valeur prescrite (11) du volume courant soit atteinte, une régulation supplémentaire d'un paramètre de ventilation caractéristique pour la ventilation du patient étant de plus effectuée et un signal de régulation correspondant étant délivré à la source de gaz respiratoire (76), **caractérisé en ce que** l'unité de commande (77) est conçue et configurée selon l'une des revendications 1 à 19, en vue de l'exécution des étapes.
